(19) Europäisches Patentamt
European Patent Office
Office européen des brevets

(11) **EP 4 235 168 B1**

(12) **EUROPEAN PATENT SPECIFICATION**

(45) Date of publication and mention of the grant of the patent:
**11.09.2024  Bulletin 2024/37**

(21) Application number: **22159238.9**

(22) Date of filing: **28.02.2022**

(51) International Patent Classification (IPC):
**G01N 27/74** *(2006.01)*      **G01N 33/543** *(2006.01)*

(52) Cooperative Patent Classification (CPC):
**G01N 33/54326; G01N 27/745**

(54) **A METHOD AND SYSTEM FOR MEASURING A MODIFIED PROPERTY OF A SAMPLE COMPRISING MAGNETIC PARTICLES IN LIQUID SUSPENSION**

VERFAHREN UND SYSTEM ZUR MESSUNG EINER MODIFIZIERTEN EIGENSCHAFT EINER PROBE, DIE MAGNETISCHE TEILCHEN IN EINER FLÜSSIGEN SUSPENSION UMFASST

PROCÉDÉ ET SYSTÈME PERMETTANT DE MESURER UNE PROPRIÉTÉ MODIFIÉE D'UN ÉCHANTILLON COMPRENANT DES PARTICULES MAGNÉTIQUES DANS UNE SUSPENSION LIQUIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**30.08.2023   Bulletin 2023/35**

(73) Proprietors:
• **Julius-Maximilians-Universität Würzburg**
  **97070 Würzburg (DE)**
• **Friedrich-Alexander-Universität Erlangen-Nürnberg**
  **91054 Erlangen (DE)**

(72) Inventors:
• **Vogel, Patrick**
  **97279 Prosselsheim (DE)**
• **Rückert, Martin**
  **97234 Reichenberg (DE)**
• **Lyer, Stefan**
  **91054 Erlangen (DE)**
• **Tietze, Rainer**
  **91054 Erlangen (DE)**
• **Alexiou, Christoph**
  **91054 Erlangen (DE)**
• **Friedrich, Bernhard**
  **91054 Erlangen (DE)**

(74) Representative: **Lucke, Andreas**
**Boehmert & Boehmert**
**Anwaltspartnerschaft mbB**
**Pettenkoferstrasse 22**
**80336 München (DE)**

(56) References cited:
**WO-A1-2021/212144**

• **KAI WU ET AL: "Magnetic particle spectroscopy-based bioassays: methods, applications, advances, and future opportunities", JOURNAL OF PHYSICS D: APPLIED PHYSICS, INSTITUTE OF PHYSICS PUBLISHING, BRISTOL, GB, vol. 52, no. 17, 18 February 2019 (2019-02-18), pages 173001, XP020340458, ISSN: 0022-3727, [retrieved on 20190218], DOI: 10.1088/1361-6463/AB03C0**
• **KAI WU ET AL: "One-step, Wash-free, Nanoparticle Clustering-based Magnetic Particle Spectroscopy (MPS) Bioassay Method for Detection of SARS-CoV-2 Spike and Nucleocapsid Proteins in Liquid Phase", ARXIV.ORG, CORNELL UNIVERSITY LIBRARY, 201 OLIN LIBRARY CORNELL UNIVERSITY ITHACA, NY 14853, 1 August 2021 (2021-08-01), XP091029448**

**Description**

FIELD OF THE INVENTION

The present invention is in the field of magnetic nanoparticle spectroscopy. More precisely, the present invention relates to the measurement of a modified property of magnetic micro- or nanoparticles in suspension using time-varying magnetic fields.

BACKGROUND

The characterization of ensembles of magnetic nanoparticles (MNP) is an ongoing task in many fields of research. Magnetic particle spectroscopy (MPS) is a quite young technology for the characterization of MNPs. It uses an oscillating magnetic field of a sufficient field strength to drive a MNP ensemble periodically into a non-linear magnetization response.

The non-linear magnetization response can reveal specific information for each MNP type encoded in higher harmonics of the excitation frequency. The magnetic moments of MNPs follow the time-varying external field directions through a combined Neel and Brownian relaxation mechanism. The Brownian relaxation process is dominant for single-core iron oxide MNPs with core sizes above 20 nm and can reflect the degree of freedom of physical rotational motion of the MNPs. When the rotational degree of freedom is impeded, the non-linear magnetization response of the MNP will be affected, typically observed as a reduction of the amplitude of the higher harmonics. This property of the non-linear magnetization response can therefore enable a measurement of sample properties influencing the rotational degree of freedom of the MNPs.

For example, when the magnetic nanoparticles are suspended in liquid, the dynamic magnetic response can be used for measuring parameters of the surrounding solution, such as the viscosity and temperature, as well as the conjugations of chemical or biological compounds on the surface of the MNPs, e.g. via a modification of the MNP's hydrodynamic diameter or clustering.

Wu et al. ("One-Step, Wash-free, Nanoparticle Clustering-Based Magnetic nanoparticle Spectroscopy Bioassay Method for Detection of SARS-CoV-2 Spike and Nucleocapsid Proteins in the Liquid Phase"; ACS Appl. Mater. Interfaces 2021, 13, 44136-44146) teach a method of detecting target proteins based on polyclonal antibodies (pAbs) surface-conjugated to MNPs, such that each MNP will be able to specifically bind to target protein molecules. Samples comprising the surface-conjugated MNPs in liquid suspension as well as the target proteins are subjected to a time-varying magnetic field, and the non-linear response of the samples are compared to reference measurements of samples without the target proteins. The presence of the target proteins can be detected based on a reduction of the amplitude of higher harmonics, which can be caused by nanoparticle clustering mediated by the target proteins. WO 2021/212144 A1 discloses a bioassay system that includes at least one conductive excitation coil, the at least one conductive excitation coil configured to generate an alternating magnetic field including a first frequency and a second frequency. The bioassay system further includes a sample mount configured to position a sample within the at least one conductive excitation coil, and at least one sensing conductive coil configured to determine a magnetic response of a sample positioned within the sample mount to the alternating magnetic field.

SUMMARY OF THE INVENTION

The known methods for measuring sample properties based on the dynamic magnetic response of magnetic nanoparticles are however usually reliant on accurate measurement control and require long acquisition times on the order of seconds to accurately resolve the signal amplitudes in addition to sophisticated data processing. This means that the measurement limits the sample analysis, but also that the drive currents during the measurement time can heat up the measurement device, which can lead to undesirable temperature drifts or dead time for cooling periods. Similar modalities, such as AC-susceptibility (ACS) measurement can overcome this issue offering a more sensitive technique to investigate and determine parameters of the environmental serum, but often hampers from the fact, that the handling of those devices as well as the data processing not only require sophisticated hardware but also specifically trained personnel.

In view of this state-of-the-art, the object of the invention is to provide a fast and robust method for sample characterization based on the dynamic magnetic response of magnetic micro- or nanoparticles.

This object is solved by two methods, a computer program and a system according to the independent claims. The dependent claims relate to preferred embodiments.

According to a first aspect, the invention relates to a method for measuring a modified property of a sample comprising magnetic particles in suspension using time variable magnetic fields as set out in independent claim 1.

The method comprises applying a time-varying magnetic excitation field, the excitation field having an excitation amplitude and an excitation frequency, and a magnetic field gradient to the sample, such that the magnetic particles are driven into a non-linear magnetization response regime, and recording a non-linear magnetization response of the sample for obtaining a signal metric indicative for a phase of a higher harmonic with respect to the excitation frequency in the non-linear magnetization response. The method further comprises determining a measurement signal indicative of the modified property, the measurement signal being based on the signal metric obtained for the sample and the same signal metric obtained for a reference sample without the modified property, the reference sample also comprising the magnetic particles in suspension.

Common MPS devices are working with a strong time-varying magnetic field $H_{AC}$, without an additional offset field. Conversely, ACS devices are working with weak drive fields $H_{AC}$, whose amplitude is small enough to result in an essentially linear response of the magnetization characteristic of the magnetic particles at the excitation frequency, generally below 2 mT, and the offset magnetic fields $H_{DC}$ (static or with low frequency with respect to the excitation frequency) excitation frequency are varied to measure the AC-susceptibility of a sample.

However, the Inventors observed that outside these two commonly measured regimes, at intermediate excitation amplitude of the time-varying magnetic excitation field and intermediate offset field, the complex signal of the higher harmonics of the excitation field experiences detectable phase shifts. These phase shifts can occur at crossing points of the real and imaginary parts of the complex signal, when plotted for a given excitation field as a function of offset magnetic field, in the following referred to as critical points. The crossing points may be located close to nodes of the signal amplitude, as predicted by the respective Chebyshev-like polynomials, and therefore at positions where, commonly, low (amplitude) signal would be expected.

However, the Inventors found that minor changes of the hydrodynamic diameter of a magnetic micro- or nanoparticle, e.g. induced by a binding event of a target analyte, can be reliably detected by tracking the phase difference between the sample and a reference sample in a wide interval around the critical point associated with the phase shift, despite the reduced signal amplitude.

The Inventors further observed that by applying a magnetic gradient field, the measurement becomes less sensitive on the exact location of the critical point for a given magnetic particle in the parameter space of offset magnetic field strength and excitation amplitude, in the following also referred to as critical point condition. Rather, it was found that the phase measurement can reliably detect minor changes of the Brownian relaxation characteristics of the magnetic particles based on the phase signal of at least one of the higher harmonics for a given gradient comprising spatially varying magnetic offset field strengths of a few mT. The Inventors found that, for small gradients, the gradient can also increase the signal-to-noise ratio of the measurement. Due to the robust nature of the measurement and since the measurement need not rely on an accurate measurement of the signal amplitude, the measurement signal can be obtained by measuring each of the sample and the reference sample once over a short measurement duration on the order of milliseconds, such that the measurement becomes suitable for fast sample characterization.

In principle, any property affecting the Brownian relaxation characteristics of the magnetic particles could be measured in this way. Additionally, the dependence of the effect on the offset magnetic field strength may also be used to measure small changes of external magnetic fields by tracking the signal metric for a sample. In other words, in some embodiments, the reference sample may be the sample measured at a different point in time, e.g. not subjected to the changed external magnetic field. However, one of the more interesting applications may be the detection of an analyte in a liquid suspension comprising the magnetic particles functionalized to bind to the analyte.

In preferred embodiments, the magnetic particles are functionalized to promote binding to a selected analyte, such that the modified property is a suspected presence of the selected analyte in the sample, wherein the analyte is in particular a biomolecule, preferably a protein, an antibody, a virus, RNA, DNA, a procariot, fungus, eucariot, or a portion thereof.

The binding of the analyte to the magnetic particles will generally change the effective hydrodynamic particle diameter, which may then influence the complex signal response of the higher harmonics, in particular by shifting the critical point condition. As a result, even small changes in hydrodynamic diameter can be detected due to the large slope associated with the phase jump at the critical point.

The magnetic particles can be prepared with functional groups to target the analytes, for example by silanizing magnetic nanoparticles using an aminosilane compound and functionalization of the resulting particle with selective binding proteins (antigen or antibodies), e.g. using suitable cross-linking agents. The resulting (functionalized) magnetic particle may selectively bind to a target biomolecule, such as a target protein of an antibody or a virus, for broad application in biochemical sensing.

In the measurement, the sample may comprise a solution associated with the suspected presence of the analyte and the reference sample may comprise a reference solution not associated with the suspected presence of the analyte, e.g. buffer solution. Both the sample and the reference sample comprise the magnetic particles functionalized to target the analyte, such that the signal metric obtained from measuring the sample and the reference sample is indicative of the suspected presence of the analyte.

The magnetic particles will generally be nano- or microparticles with a size between about 5 nm and about 10

μm, preferably with a sub-micron magnetic core, wherein the diameters of magnetic particle crystallites are such that the magnetic response is essentially superparamagnetic. For example, the magnetic particles may have a magnetic core with a diameter between about 5 nm and 1 μm and in particular between about 1 nm and about 300 nm, preferably between about 5 nm and 100 nm, and may have a shell, which may be nonmagnetic and which is functionalized to bind to the target analyte.

According to said first aspect, the magnetic field gradient and the time-varying magnetic excitation field are selected such that a range of offset magnetic field strengths of the magnetic field gradient overlaps a critical offset magnetic field range associated with a phase jump of the higher harmonic, when the sample and/or the reference sample is subjected to the excitation field.

Around the critical point condition, the phase of the higher harmonic may vary significantly, which results in an increased sensitivity to changes of the sample properties. In particular, changes of the effective hydrodynamic particle diameter may shift the critical point conditions, resulting in a large change of the phase close to the critical point.

In preferred embodiments, the phase of the higher harmonic jumps by substantially 180° in the critical offset magnetic field range.

Generally, the phase jump associated with a critical point will have a magnitude of around 180°. However, the largest slope of the phase as a function of offset magnetic field strength may be in a range smaller than the range associated with the full phase of 180°, such as a critical offset magnetic field range in which the phase jumps by about 150°, or by about 175°. Accordingly, for an optimal sensitivity, the critical offset magnetic field range should be the range in which the majority of the phase jump is located, or a portion thereof, such that the slope of the phase as a function of offset magnetic field strength is large and a larger signal can be measured. On the other hand, a combination of excitation field and offset magnetic field gradient, which define a condition offset from the critical point may increase the dynamic range of the measurement in embodiments.

In preferred embodiments, the critical offset magnetic field range is centered on a crossing point of the real and imaginary components of the non-linear magnetization response associated with the higher harmonic.

The location of the phase jump may be determined by the crossing point of the real and imaginary components of the higher harmonic for a given excitation field, when the real and imaginary components of the higher harmonic are plotted as a function of offset magnetic field strength. The real and imaginary components of the higher harmonic may follow respective Chebyshev-like polynomials for the higher harmonic, which predict that higher harmonics of different order may have critical points located at different offset magnetic field strengths. Hence, depending on a combination of a given type of magnetic particles, a given excitation field and a given magnetic field gradient, different orders of the higher harmonics may provide a large measurement signal, for which the critical points are located in or close to the range of offset magnetic field strengths.

In some embodiments, the method may comprise selecting a specific higher harmonic for determining the measurement signal. However, since the complex amplitudes of multiple higher harmonics can be determined during digital processing of a recorded non-linear magnetization response, the selection of the higher harmonic may also be based on the measurement signal. For example, the higher harmonic associated with the highest sensitivity or the largest measurement signal may be empirically selected, or the modified property may be measured based on the measurement signals of multiple higher harmonics.

In preferred embodiments, the method comprises processing the non-linear magnetization response of the sample for obtaining complex signal amplitudes of the higher harmonics, and the signal metric is based on the complex signal amplitudes of the higher harmonic.

For example, the method may record the non-linear magnetization response as a digitized analog signal, and the digitized analog signal may be processed according to a sine and cosine transformation of a selection of higher harmonics, e.g. the first 10 or 20 higher harmonics or a select one of the higher harmonics. Based on the complex signal amplitudes of the higher harmonics, the amplitude and phase of the higher harmonics may be calculated.

In preferred embodiments, processing the non-linear magnetization response comprises a Fourier transform of the non-linear magnetization response.

The Fourier transform of the non-linear magnetization response may reveal the complex signal amplitudes of the higher harmonics and may implement the sine and cosine transformation.

Preferably, the signal metric is based on the phase determined for the higher harmonic.

In preferred embodiments, the signal metric is proportional to or mathematically equivalent to a phase of the higher harmonic.

As discussed above, the method may exploit an increased sensitivity to changes in the sample at the critical point(s), which is associated with a phase jump, such that the phase can be an effective signal metric for detecting sample changes affecting the non-linear magnetization response of the magnetic particles. However, since the crossing point is usually close to a node of the signal amplitude, at which large relative changes of the amplitude are expected, the measurement signal may also be determined based on a function of the complex signal amplitudes, e.g. by including a measurement signal contribution of the amplitude and the phase. In other words, the measurement signal may merely be indicative for a phase difference of the higher harmonic for the sample and for the reference sample.

The condition for the offset magnetic field strength at which a critical point is located may depend on the components of the magnetic field gradient oriented along the direction of the excitation field.

In preferred embodiments, the excitation field induces a magnetic field having an excitation field direction, and wherein a parallel component of the magnetic field gradient along the excitation field direction varies over a measurement volume associated with the sample and/or the reference sample.

By varying the offset magnetic field strength over the measurement volume, the critical point condition may be fulfilled in at least a portion of the sample or the reference sample.

In preferred embodiments, the magnetic field gradient comprises offset magnetic field strengths larger than 2 mT.

The inventors found in their experiments with iron oxide magnetic particles that the phase jumps are observed for offset magnetic field strengths above 2 mT, preferably larger than 2.5 mT. Although a detectable measurement signal should also be present at lower offset magnetic fields according to the simulations of the Inventors, offset magnetic field strengths above 2 mT may facilitate implementing the method without sophisticated measurement hardware. As an example, the offset magnetic field strengths of the magnetic field gradient in the measurement volume may be centered on a value between about 2 mT and about 40 mT, such as between 2 mT and 20 mT or between 2 mT and 10 mT.

The range of offset magnetic field strengths of the magnetic field gradient in the measurement volume may be selected based on a desired compromise between a robustness of the method with respect to the critical point conditions and a magnitude of the measurement signal, which may benefit from a smaller width of the magnetic field gradient. As an example, the range of offset magnetic field strengths of the magnetic field gradient in the measurement volume may be on the order of between about 0.1 mT to about 5 mT, such as about 0,5 mT, about 1 mT, or about 2 mT.

In preferred embodiments, applying the magnetic field gradient comprises applying the field of a permanent magnet.

The permanent magnet may generate a substantially static magnetic field gradient in the measurement volume and may contribute to the robustness of the method. The permanent magnet may be offset from the measurement volume, in particular offset in a direction perpendicular to the direction of the excitation field, for inducing a suitable gradient across the measurement volume. The permanent magnet may induce offset magnetic fields, which need not align with excitation direction associated with the time-varying magnetic excitation field. The skilled person will appreciate that in these cases, the critical point condition is substantially selected based on the magnetic field components of the magnetic field gradient parallel to the excitation direction. The offset fields may further be tuned using an additional homogenous magnetic field, e.g. applied with a magnetic field coil, in order to select the critical point condition for the sample and/or the reference sample.

In preferred embodiments, the excitation field has an excitation amplitude larger than 2 mT.

Similar to the value of the offset magnetic field strength for the critical point condition, the Inventors observed a strong measurement signal in their experiments for critical point conditions at excitation amplitudes, which are larger than 2 mT, preferably larger than 2.5 mT. In particular, the excitation amplitudes may be outside of the linear range of the magnetization characteristic of the magnetic particles. The simulations of the Inventors also predict a measurement signal for lower excitation amplitudes. However, based on the empirical measurement results, an excitation field amplitude above about 2 mT may facilitate implementing the method without sophisticated measurement hardware.

In preferred embodiments, the measurement signal is mathematically equivalent or proportional to a difference between the signal metric obtained for the sample and the signal metric obtained for the reference sample.

For example, the phase of the higher harmonics may be recorded for the reference sample and the sample, and the resulting signal metrics may be subtracted from each other as a phase difference between a higher harmonic of the sample and of the reference sample.

In preferred embodiments, the method further comprises applying the excitation field and the magnetic field gradient to the reference sample without the modified property, and recording a non-linear magnetization response of the reference sample for obtaining the signal metric of the higher harmonic for the reference sample.

The sample and the reference sample may be measured in parallel or in succession for obtaining respective signal metrics for determining the measurement signal indicative of the modified property. However, the reference sample may also be measured and the resulting signal metric may be recorded and compared to respective signal metrics of a plurality of different samples in sample characterization applications measured at different points in time.

The measurement signal may be determined on a processing system. The processing system may comprise a single processing unit or may comprise a plurality of processing units, which may be functionally connected. The processing units may comprise a microcontroller, an ASIC, a PLA (CPLA), an FPGA, or other processing device, including processing devices operating based on software, hardware, firmware, or a combination thereof. The processing devices can include an integrated memory, or communicate with an external memory, or both, and may further comprise interfaces for connecting to sensors, devices, appliances, integrated logic circuits, other controllers, or the like, wherein the interfaces may be configured to receive or send signals, such as electrical signals, optical signals, wireless signals, acoustic signals, or the like. For example, the processing system may be connected to an alternating current generator for driving an excitation coil to apply the time-varying magnetic field and/or may be coupled to an analog to digital converter for recording a current induced in a measurement coil to record the non-linear magnetization response of the sample. The processing system may also be configured to determine the signal metric of the higher harmonic based on the recorded non-linear magnetization response, e.g. by implementing a fast Fourier transform of the recorded non-linear magnetization response.

According to a second aspect, the invention relates to a further method for measuring a modified property of a sample comprising magnetic particles in suspension using time variable magnetic fields as set out in independent claim 9.

The method comprises applying a time-varying magnetic excitation field, the excitation field having an excitation amplitude and an excitation frequency, and a variable offset magnetic field to the sample, such that the magnetic particles are driven into a non-linear magnetization response regime, and recording a non-linear magnetization response of the sample for obtaining a signal metric indicative for a phase of a higher harmonic with respect to the excitation frequency in the non-linear magnetization response. The method further comprises determining a measurement signal indicative of the modified property, the measurement signal being based on the signal metric obtained for the sample and the signal metric obtained for a reference sample without the modified property, the reference sample also comprising the magnetic particles in suspension.

According to said second aspect, the variable offset magnetic field is selected from a critical offset magnetic field range associated with a phase jump of the higher harmonic, when the sample and/or the reference sample is subjected to the excitation field.

Hence, the method may also be implemented by selecting the critical point condition using an excitation field having a suitable excitation amplitude and a suitable non-zero offset magnetic field. The resulting method may be more susceptible to instrumentation noise and may require more sophisticated measurement hardware, but may also attain a higher measurement signal, as the critical point condition may be fulfilled in a larger portion of the sample volume, when compared to the use of a comparatively large magnetic field gradient.

The offset magnetic field may be generated with a magnetic field coil. The offset magnetic field may be substantially static when compared to the excitation frequency and/or when compared to an acquisition time of the non-linear magnetization response.

In preferred embodiments, the offset magnetic field and/or the excitation amplitude are varied across the phase jump of the higher harmonic for the sample and/or the reference sample, while recording the non-linear magnetization response of the sample to the time-varying magnetic excitation field, wherein the measurement signal is preferably based on a resulting trace of the signal metric for the sample and a corresponding trace of the signal metric for the reference sample.

In other words, the critical point condition may be scanned by varying the excitation amplitude and/or the offset magnetic field in order to select the critical point condition. For example, the offset magnetic field maybe varied in steps while the sample is subjected to the excitation field, for recording the nonlinear magnetization response close to a critical point condition of the higher harmonic. After the characterization, it may be sufficient to measure a single value of the signal metric for the sample and the reference sample.

The method according to the second aspect may be combined with the method according to the first aspect, e.g. may comprise applying a magnetic field gradient in addition to a variable offset magnetic field, such as a variable offset magnetic field generated with a magnetic field coil for tuning the measurement system. Further, the method according to the second aspect may also benefit from any feature of the preferred embodiments of the first aspect.

According to a third aspect, the invention relates to a measurement system for measuring a modified property of a sample comprising magnetic particles in suspension using time variable magnetic fields as set out in independent claim 11.

The system comprises an inductive sensor associated with a measurement volume, a magnetic field coil for generating a time-varying magnetic field in the measurement volume, and an offset magnetic field source for generating an offset magnetic field in the measurement volume, and a control system. The control system is configured to drive the magnetic field coil to apply a time-varying magnetic excitation field to the measurement volume, the excitation field having an excitation amplitude and an excitation frequency, such that the magnetic particles of the sample are driven into a non-linear magnetization response regime, and receive a non-linear magnetization response of the sample from the inductive sensor for obtaining a signal metric indicative for a phase of a higher harmonic with respect to the excitation frequency in the non-linear magnetization response. The control system is further configured to determine a measurement signal indicative of the modified property, the measurement signal being based on the signal metric obtained for the sample and the signal metric obtained for a reference sample without the modified property, the reference sample also comprising the magnetic particles in suspension.

The system may implement the method according to the first aspect or the second aspect or any combination of their embodiments.

According to said third aspect, the offset magnetic field and the time-varying magnetic excitation field are selected, such that the offset magnetic field is in a critical offset magnetic field range associated with a phase jump of the higher harmonic, when the sample and/or the reference sample is subjected to the excitation field.

In preferred embodiments, the magnetic field source comprises a variable magnetic field generator for generating the offset magnetic field in the critical offset magnetic field range.

The variable magnetic field generator may be a constant current source coupled to a coil for inducing an offset magnetic field in the measurement volume. The variable magnetic field generator may generate a substantially homogeneous magnetic field in the measurement volume for selecting the critical point condition in a large portion of the sample volume.

In preferred embodiments, the variable magnetic field generator is coupled to the magnetic field coil or to a second magnetic field coil.

In preferred embodiments, the variable magnetic field generator is configured to vary the offset magnetic field over the critical offset magnetic field range for the sample and the reference sample, while the system records the non-linear magnetization response to the time-varying magnetic excitation field.

In preferred embodiments, the magnetic field source is adapted to induce a magnetic field gradient in the measurement volume and in particular comprises a permanent magnet arranged to induce a magnetic field gradient in the measurement volume.

The magnetic field gradient induced by the permanent magnet may comprise the offset magnetic field in the critical offset magnetic field range.

However, the skilled person will appreciate that a magnetic field gradient may also be implemented dynamically using suitable magnetic field coils, or magnetic field coils and permanent magnets may be combined to implement a range of offset magnetic fields overlapping the critical offset magnetic field range.

In preferred embodiments, the inductive sensor comprises a measurement coil, in particular a gradiometer.

The gradiometer may comprise two magnetometers in series for measuring a magnetic flux difference between the magnetometers, and the sample may be arranged in one of the magnetometers during a measurement. The gradiometer may reduce a contribution of the excitation field in the recorded magnetization response and may therefore simplify an acquisition hardware. However, the system may equally reduce a corresponding signal contribution using measurement hardware, e.g. using suitable high-pass filtering.

According to a fourth aspect and as set out in independent claim 15, the invention relates to a non-transitory medium comprising machine-readable instruction, which, when executed by a processing system of a measurement system according to the third aspect, implement a method according to the first aspect and/or the second aspect.

DETAILED DESCRIPTION OF EMBODIMENTS

The features and numerous advantages of the methods and system according to the present invention will best be understood from a detailed description of preferred embodiments with reference to the accompanying drawings, in which:

| | |
|---|---|
| Fig. 1 | schematically illustrates the non-linear magnetization response of a sample comprising magnetic nanoparticles in liquid suspension; |
| Fig. 2A, 2B | illustrate an example of the real and imaginary parts as well as the phase of the third harmonic as a function of offset magnetic field strength; |
| Fig. 3A | shows an example of the measured real and imaginary parts as well as the phase of the third harmonic as a function of offset magnetic field strength; |
| Fig. 3B | illustrates the phase of the third harmonic as a function of both excitation field amplitude and offset magnetic field strength in a grey-scale color plot; |
| Fig. 4 | illustrates an example of a method for measuring a modified property of a sample comprising magnetic nanoparticles in suspension using time variable magnetic fields; |
| Fig. 5 | illustrates a system for implementing the method of Fig. 4; |
| Fig. 6 | illustrates an example of a nanoparticle functionalization sequence for targeting a specific analyte, based on a SARS CoV-2-S1 protein; |
| Figs. 7A-D | illustrate example measurements of the phase difference of the third harmonic between two pairs of samples; |

Figs. 8A, 8B    illustrate examples of measurements of the phase difference between two respective pairs of samples as a function of offset magnetic field and excitation field amplitude for a plurality of different higher harmonics;

Fig. 9A    schematically illustrates an example of a system similar to the system of Fig. 5, further comprising a permanent magnet for inducing a magnetic field gradient in the measurement volume;

Fig. 9B    schematically illustrates an example of a distribution of the offset magnetic field components projected onto the excitation field direction in a system as shown in Fig. 9A;

Fig. 10    illustrates an example of a method for measuring a modified property of a sample comprising magnetic nanoparticles in suspension using a magnetic field gradient;

Figs. 11A, 11B    illustrate raw data of measurements using a system as illustrated in Fig. 9A based on a method as illustrated in Fig. 10; and

Figs. 12A-C    illustrate measurements of the presence of an analyte at different concentrations based on the phase difference of the 7th and 9th higher harmonics as well as based on fluorescence activated cell sorting, respectively.

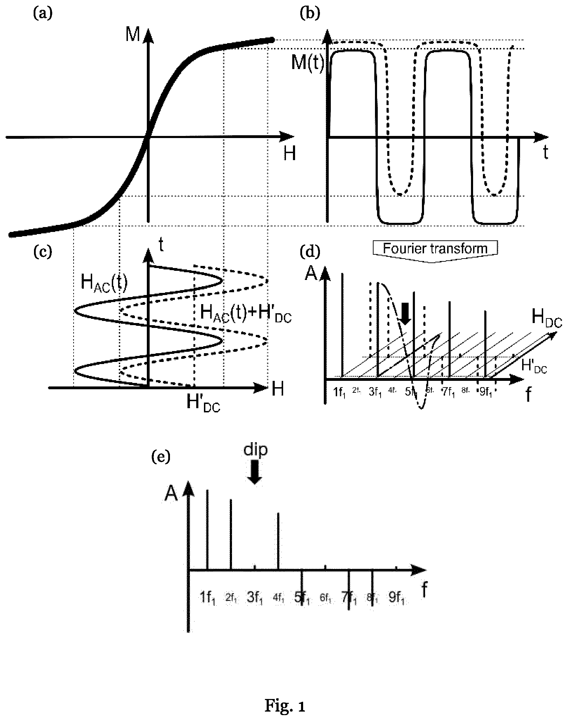

Fig. 1 schematically illustrates the non-linear magnetization response of a sample comprising magnetic nanoparticles in liquid suspension, such as iron oxide nanoparticles with a crystallite diameter between about 5 nm and about 100 nm in a liquid suspension.

In absence of an external magnetic field, all magnetic nanoparticles in such a sample may be statistically oriented, which may cause the magnetization of the sample to be zero. Increasing the external magnetic field strength can lead to more and more particles aligning along the external field and cause an increase of the magnetization. At a specific magnetic field strength $M_{sat}$, all particles may be aligned and the magnetization of the sample is saturated (saturation magnetization).

The dependency of the sample magnetization $M$ to the external magnetic field strength H may be effectively superparamagnetic and can be described by the Langevin function $L\ (\xi)$:

$$L(\xi) = \coth(\xi) - \frac{1}{\xi} \ \text{ with } \xi = \frac{\mu_0 m H}{k_B T} \ , \hspace{3cm} (1)$$

with m as the magnetic moment of a particle, $\mu_o$ as the vacuum permeability, $k_B$ as the Boltzmann constant and T as temperature. The resulting curve is shown in subfigure Fig. 1(a), showing a substantially linear response regime around zero external magnetic field $H$ as well as a saturation regime, in which the slope of the magnetization with respect to the external magnetic field strength $H$ is reduced.

When the sample is subjected to a time-varying field $H_{AC}(t)$ with an excitation frequency $f_{AC}$ and a substantially static offset magnetic field $H_{DC}$ with sufficient amplitude, the magnetic nanoparticles can be driven into a non-linear response regime, in which the magnetization M(t) is no longer a linear function of the external magnetic field strength N.

Subfigures Fig. 1(c) and Fig. 1(b) show two different driving fields with identical sinusoidal time-varying field components $H_{AC}(t)$ components and the resulting time dependent magnetization response, respectively, for the case of zero offset magnetic field (solid curves), and non-zero offset magnetic field $H_{DC}'$ (dashed curves). Dashed lines between subfigures Fig. 1(a)-(c) illustrate the magnitude of the magnetic field strength and of the resulting magnetization response in relation to the magnetization curve in Fig. 1(a).

As can be seen from Fig. 1(b), the magnetization response M(t) of the sample to a time-varying field $H_{AC}(t)$ ($H_{AC}(t)+ H_{DC}'$) with a sufficient magnitude differs from a sinusoidal shape. Rather, the magnetization response the magnetic nanoparticles becomes non-linear and exhibits higher harmonics of the excitation frequency $f_{AC}$ in frequency domain.

Fig. 1(d) shows an example of a Fourier spectrum of the signals of Fig. 1(b). The horizontal axis is frequency, the vertical axis shows the amplitude of the real part of the Fourier transform A of the magnetization response, and the perpendicular axis indicates offset magnetic field $H_{DC}$. Fig. 1(e) shows the dependency of the real part of the Fourier transform A as a function of frequency for the example with offset magnetic field $H_{DC}'$ (corresponding to the dashed curves of Figs. 1(b)-d)).

As can be seen in the Fourier spectrum associated with offset magnetic field $H_{DC}'$ illustrated in Fig. 1(e), the magnetization response M(t) consists not only of the fundamental frequency but also odd (and even) higher harmonics

($f_n = n \cdot f_1$) depending on the presence of an offset magnetic field HDC, which can be visualized in the Fourier spectrum.

The Fourier spectrum further shows a complex distribution of the signal amplitude among the different higher harmonics, where in the exemplified example, the third harmonic, which can be measured at frequency $3 \cdot f_{AC}$ ("$3f_1$"), shows a signal amplitude close to zero ("dip"). The amplitude $A_n$ of the higher harmonics for a given time-varying excitation field with excitation amplitude $H_{AC}$ as a function of offset magnetic field $H_{DC}$ strength may be predicted using the Chebyshev polynomials according to

$$A_n(H_{DC}) = -\frac{2\mathrm{i}}{\mathrm{T}} M'(H_{DC}) * \left( U_{n-1}\left(\frac{H_{DC}}{H_{AC}}\right) \cdot \sqrt{1 - \left(\frac{H_{DC}}{H_{AC}}\right)^2} \right) \tag{2}$$

where $U_{n-1}$ represents the Chebyshev polynomials of the second kind and $M'(H_{DC})$ is the derivative of the magnetic response, which is exemplified for the third order higher harmonic by the curve in Fig. 1(d) with intermittent dashes (- · -).

In the signal, the 'dips' within a Fourier spectrum of a MPS signal may be understood as a destructive interference of two separate signal components $S_{pos}$ and $S_{neg}$ associated with respective switches of the magnetization in opposite directions, which arise from the time signal S(t)=dM(t)/dt, which is captured as an induction signal by a receive coil. At the condition of the dips $H_{DC}$, the signals $S_{pos}$ and $S_{neg}$ associated with a higher harmonic may have similar amplitude, but may effectively cancel each other due to a phase shift between the signals $S_{pos}$ and $S_{neg}$. Said phase shift may be offset magnetic field dependent due to a shift of the points in time at which the sample is not in a saturation regime (as can be seen from subfigure Fig. 1 (b)). As a result of the interference, the phase of the higher harmonic signal may experience a phase jump close to the conditions for the "dip".

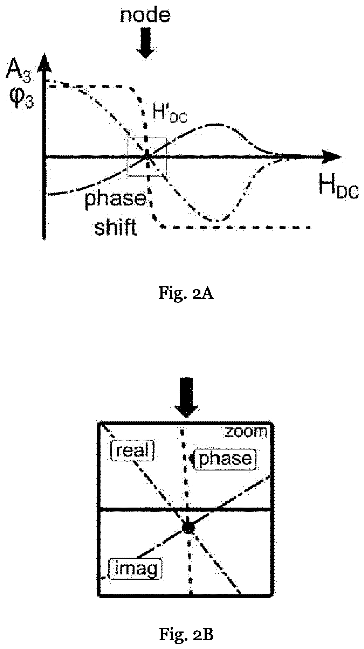

Fig. 2A illustrates an example of the real and imaginary parts of the amplitude $A_3$ as well as the phase $\varphi_3$ of the third harmonic as a function of offset magnetic field strength $H_{DC}$. Fig. 2B illustrates a zoom of the square portion of Fig. 2A around the offset magnetic field strength $H_{DC}$'.

As can be seen from the diagram, the phase $\varphi_3$ of the third harmonic shifts by 180° at the crossing of the real part and the imaginary part of the amplitude. The crossing is close to, but shifted from the nodes (zeros) of the real part and the imaginary part of the amplitude $A_3$. The slope of the phase $\varphi_3$ with respect to offset magnetic field strength $H_{DC}$ is larger than the slope of the real part and the imaginary part of the amplitude $A_3$ between respective extremal values.

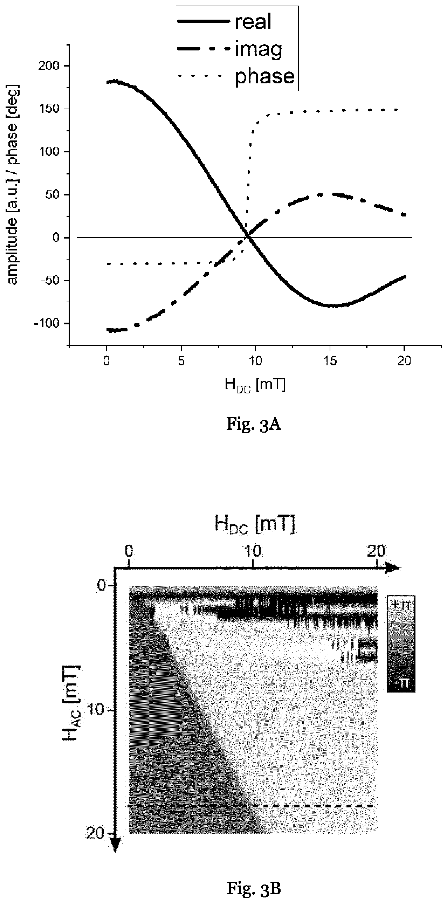

Fig. 3A shows an example of the measured real and imaginary parts of the amplitude $A_3$ as well as the phase $\varphi_3$ of the third harmonic in an experiment with functionalized magnetic iron oxide nanoparticles in liquid suspension, as a function of offset magnetic field strength $H_{DC}$. Fig. 3B illustrates the phase $\varphi_3$ of the third harmonic as a function of both excitation field amplitude $H_{AC}$ and offset magnetic field strength $H_{DC}$ in a grey-scale color plot, wherein the curve in Fig. 3B is highlighted as a dashed line through the visualization.

As can be seen from the measured data, the phase $\varphi_3$ shifts abruptly by substantially 180° over an offset magnetic field range of about 2,5 mT, in accordance with the theoretical prediction at the crossing of the real part and the imaginary part of the amplitude (critical point condition). The phase jump is pronounced in the data shown in Fig. 3B, starting at excitation field amplitudes $H_{AC}$ of about 2 mT and offset magnetic field $H_{DC}$ of at least 1.5 mT, and may be clearly identified for critical point conditions with excitation field amplitudes $H_{AC}$ above about 2 mT and offset magnetic fields $H_{DC}$ above about 2.5 mT. The excitation field amplitude $H_{AC}$ is preferably greater than the offset magnetic field $H_{DC}$.

The Inventors propose to exploit the strong slope associated with the phase shift around the critical point condition for these intermediate values of excitation field amplitudes $H_{AC}$ and offset magnetic field $H_{DC}$ as a sensing platform for detecting minor changes in sample properties affecting the effective hydrodynamic diameter of the magnetic nanoparticles, in particular to sense binding events to the magnetic nanoparticles.

In a liquid suspension, the magnetization response *m(t)* not only depends on the magnetic properties of the magnetic nanoparticles particles, but also on the effective viscosity experienced by the magnetic nanoparticles in the liquid medium. Specifically, the magnetization response m to an external magnetic field *H* in a liquid medium may be described by

$$\frac{d\vec{m}}{dt} = \frac{1}{\zeta}(\vec{m} \times \vec{H}) \times \vec{m} + \sqrt{\frac{2k_B T}{\zeta}} \vec{\lambda} \times \vec{m}, \tag{3}$$

with $\zeta$ as the Stokes-Einstein diffusion coefficient (viscosity-coefficient) and $\vec{\lambda}$ as normally distributed random vectors with expectation value $\langle \lambda_i(t) \rangle = 0$, $k_B$ as the Boltzmann constant and T as the temperature. The diffusion coefficient may be approximated according to $\zeta = \kappa \eta R^3$ and may depend on the viscosity $\eta$ of the surrounding liquid, the particle diameter $R$ and a shape factor $\kappa$.

Hence, a binding event to the magnetic nanoparticle, which may affect the effective particle diameter, may shift the critical point condition of the higher harmonics, which may be tracked based on the complex signal of the higher harmonics, in particular a signal metric based on the phase (e.g. the phase $\varphi_3$) of the higher harmonic.

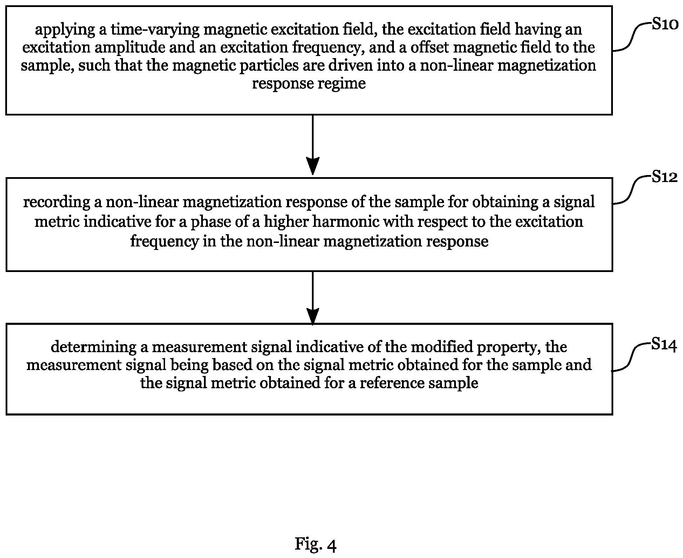

Fig. 4 illustrates an example of a method for measuring a modified property of a sample comprising magnetic particles in suspension using time variable magnetic fields. The method comprises applying a time-varying magnetic excitation field $H_{AC}(t)$, the excitation field having an excitation amplitude $H_{AC}$ and an excitation frequency $f_{AC}$, and a offset magnetic field $H_{DC}$ to the sample, such that the magnetic particles are driven into a non-linear magnetization response regime (S10), and recording a non-linear magnetization response of the sample for obtaining a signal metric indicative for a phase of a higher harmonic with respect to the excitation frequency $f_{AC}$ in the non-linear magnetization response (S12). The method further comprises determining a measurement signal indicative of the modified property, the measurement signal being based on the signal metric obtained for the sample and the signal metric obtained for a reference sample (S14).

The offset magnetic field $H_{DC}$ is selected from a critical offset magnetic field range associated with a phase jump of the higher harmonic, when the sample and/or the reference sample is subjected to the excitation field, which may generally correspond to the afore-mentioned critical point condition. The critical offset magnetic field range may be a range of offset magnetic fields $H_{DC}$ around the critical point condition, in which the phase jumps by substantially 180°, such as by 120° more, or 160° or more, and the method may therefore be sensitive to small changes of the particle parameters. However, the skilled person will appreciate that any sub-range of the critical offset magnetic field range may equally be used to implement the method in some embodiments. The reference sample also comprises the magnetic particles in suspension, but does not feature the modified property.

The (reference) sample may be characterized as in Fig. 3A/3B for identifying the critical point condition, such that further samples may be measured with substantially fixed values of the offset magnetic field $H_{DC}$ and the excitation amplitude $H_{AC}$. Additionally or alternatively, one or both of the offset magnetic field $H_{DC}$ and the excitation amplitude $H_{AC}$ may be varied to obtain curves of the phase as a function of offset magnetic field $H_{DC}$ and/or excitation amplitude $H_{AC}$ for each sample. A comparison of the measured signal metrics for the reference sample and for the sample may then indicate the (suspected) modified property.

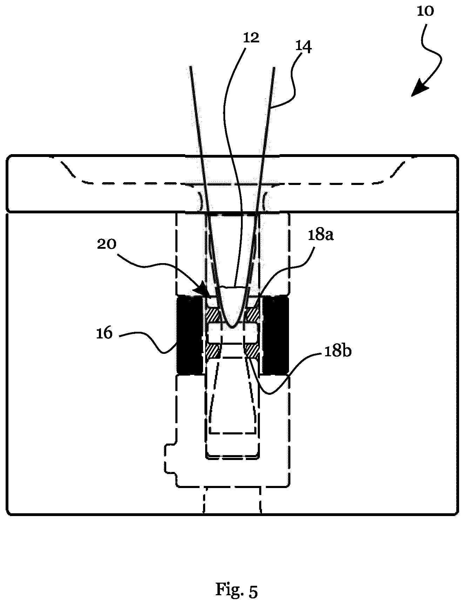

Fig. 5 illustrates a system 10 for measuring a (reference) sample 12 in a liquid container 14. The system 10 comprises an excitation field coil 16 and a detection coil 18a, 18b implemented as a gradiometer comprising winding sections 18a, 18b of opposite winding direction. The signals induced in the two winding sections 18a, 18b having opposite winding directions from the excitation coil 16 may cancel each other, when the resulting added signal is measured. Conversely, signals from (small) magnetization changes of the sample 12 in a measurement volume 20 associated with one of the winding sections 18a are not cancelled. In other words, the current of the winding sections 18a, 18b may be proportional to a magnetic flux difference between the one of the winding sections 18a and the other winding section 18b, while common magnetic flux components may be cancelled. Accordingly, small induction signals from the sample 12 may be measured without sophisticated measurement hardware.

The excitation field coil 16 may be driven with a combination of static and time-varying currents for generating the offset magnetic field $H_{DC}$ and the excitation field $H_{AC}(t)$, but some embodiments of the system 10 may also comprise different coils 16 for generating the offset magnetic field $H_{DC}$ and the excitation field $H_{AC}(t)$, respectively.

Multiple sample containers 14 may be prepared with different (reference) samples 12 and may be swapped for different measurements, during which the current sample 12 comprising the magnetic particles is subjected to the offset magnetic field $H_{DC}$ and the excitation field $H_{AC}(t)$, while recording the non-linear magnetization response from the measurement volume 20. The signal from the detection coil 18a, 18b may be amplified and digitized, and a Fourier transform of the digitized non-linear magnetization response may be used to obtain the complex amplitudes of the higher harmonics.

To measure a presence of a specific analyte in the sample 12, magnetic nanoparticles may be functionalized, such that they bind selectively to the specific analyte. The binding of the analyte to the magnetic nanoparticles may be associated with a corresponding change in the hydrodynamic diameter of the magnetic nanoparticles (MNPs), and may therefore be associated with a measurable change of the critical point condition.

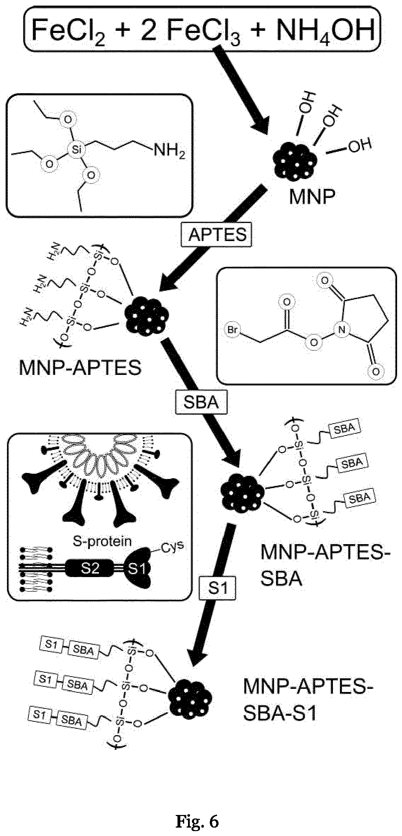

Fig. 6 illustrates an example of a nanoparticle functionalization strategy for targeting a specific analyte, based on a SARS CoV-2-S1 protein. After alkaline precipitation of the raw particles, the exemplary sequence comprises an initial silanization step, in which MNPs may be coated with APTES ((3-aminopropyl) triethoxysilane). The MNPs produced in this way may have a hydrodynamic diameter of about 200 nm with the single crystallites of the magnetic nanoparticle core showing a diameter of about 12 nm.

SARS-CoV-2-S1 protein (SARS-CoV-2 (2019-nCoV) spike S1-His, Sino Biological, China) can be covalently bound to the surface of the particles by binding SBA (N-succinimidyl bromoacetate) over cysteines present in the protein.

In the experiments of the Inventors, a 0.05 M borate buffer with pH 8.5 was used for binding. The particle concentration during processing was adjusted to 1 mg Fe/ml and 20 mM SBA dissolved in Dimethylformamide was added. The samples 12 were shaken for 2 h at 1400 rpm. After that, the particles were washed several times with buffer solution. The obtained particles were redispersed in borate buffer for binding of SARS-CoV-2-S1 protein with a concentration of 10 $\mu$g S1 per 100 $\mu$g Fe. The samples 12 were shaken again for 2 h at 1400 rpm and then washed several times with doubly distilled H2O. After binding and the last washing step, the hydrodynamic size of the particles was 330 nm and the particles were stored in doubly distilled water.

The SARS-CoV-2-S1 protein may selectively bind to SARS-CoV-2-S1 antibody as an analyte, and the magnetic nanoparticles in a sample 12 may therefore be used to detect the presence of the analyte through a change of the hydrodynamic diameter associated with the analyte bound to the magnetic nanoparticles.

For the following example measurements, for each (reference) sample 12, 25 $\mu$L of MNP-APTES-S1 dispersions (100 $\mu$g Fe/mL) was added in an 0.5 mL Eppendorf cap. This magnetic nanoparticle solution was combined with a buffer solution (reference sample), an S1 binding antibody (SARS-CoV-2-S1 antibody) solution (binding sample, S+) or a non-binding antibody (MERS-CoV-S1 antibody) solution (non-binding sample, S-), and measured without any further incubation time. The (reference) samples 12 are excited with an excitation current featuring a time-varying modulation at an excitation frequency of about 20 kHz applied to the excitation coil 16 and the non-linear magnetization response from the sample 12 may be recorded in a detection coil 18a, 18b, amplified, and digitized for extracting the complex signal amplitudes associated with the higher harmonics.

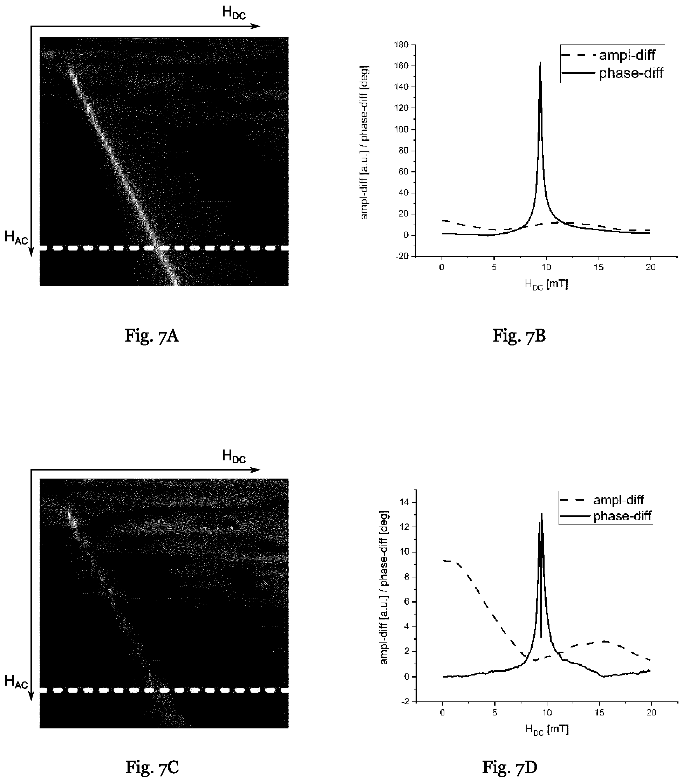

Figs. 7A-D illustrate example measurements of the phase difference $|\delta\varphi|$ of the third harmonic between two pairs of samples 12 as a function of offset magnetic field $H_{DC}$. Figs. 7A, 7C show the dependency on excitation field amplitude $H_{AC}$ in a greyscale color plot, whereas Figs. 7B, 7D, show line traces at fixed excitation field amplitude $H_{AC}$ along the white dashed lines. The axes of the greyscale color plot correspond to the axes of the measurement shown in Fig. 3B, i.e. the offset magnetic field $H_{DC}$ is varied along the horizontal axis from 0 to 20 mT (from left to right), and the excitation amplitude $H_{AC}$ is varied along the vertical axis from 0 to 20 mT (from top to bottom). The magnitude of the phase difference $|\delta\varphi|$ in Figs. 7B, 7D is in the same arbitrary units, and may therefore be compared quantitatively. Figs. 7A, 7B show the phase difference $|\delta\varphi|$ between a binding sample 12 and a reference sample 12, while Figs. 7A, 7B show the phase difference measured between two reference samples 12.

As shown in Fig. 7B, the phase difference $|\delta\varphi|$ measured between the binding sample 12 and the reference sample 12 shows a pronounced peak at an offset magnetic field strength $H_{DC}$ of about 9 mT, which may be attributed to a shift of the critical point condition caused by the presence of the binding antibody. This peak may be attributed to a modified effective hydrodynamic diameter of the magnetic nanoparticles in the sample 12 and the phase difference at the magnetic field conditions of the peak may therefore be used to detect a suspected presence of the analyte.

As can be seen from Figs. 7A, the presence of the analyte can be detected based on the phase difference $|\delta\varphi|$ for a variety of combinations of excitation field amplitude $H_{AC}$ and offset magnetic field strength $H_{DC}$. Specifically, for the third harmonic the phase difference $|\delta\varphi|$ is associated with a visible peak around critical point conditions with an excitation field amplitude $H_{AC}$ above around 2.5 mT and an offset magnetic field strength $H_{DC}$ above around 2 mT, corresponding substantially to the parameters associated with a detectable phase jump in Fig. 3B.

As shown in Fig. 7D, the same measurements also result in a peak of the phase difference $|\delta\varphi|$ at substantially the same magnetic field conditions, when two reference samples 12 are compared, which the Inventors attribute to systematic errors and instrumentation noise. However, the magnitude of the phase difference $|\delta\varphi|$ visible in Figs. 7B, 7D differs by an order of magnitude from the phase difference $|\delta\varphi|$ measured between the binding sample 12 and the reference sample 12, indicating that a corresponding measurement can be used to reliably detect the presence of the binding antibody.

Each measurement of the non-linear magnetization response may in principle be analyzed for obtaining the complex amplitudes and phases of a plurality of higher harmonics, and the signals associated with each of the higher harmonics may in principle be exploited for detecting the presence of the analyte (e.g. target antibody).

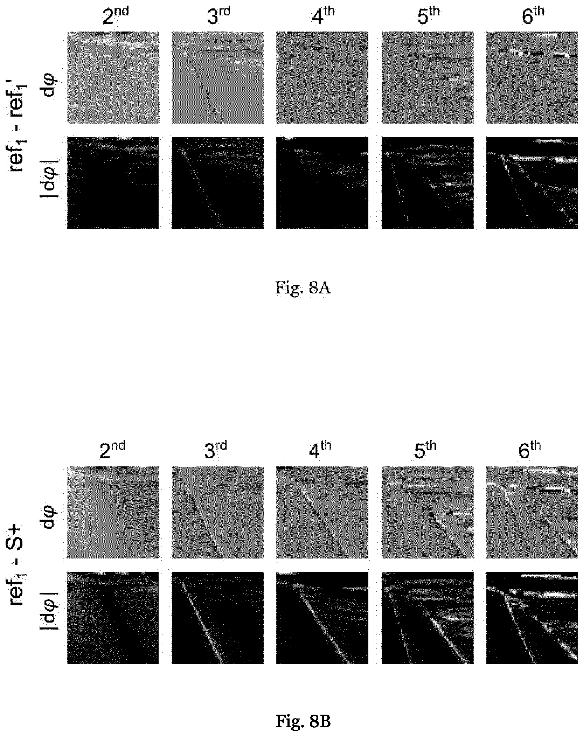

Figs. 8A, 8B illustrate examples of measurements of the phase difference $|\delta\varphi|$ between two samples 12 as a function of offset magnetic field strength $H_{DC}$ and excitation field amplitude $H_{AC}$ for a plurality of different higher harmonics. Fig. 8A shows the measured phase difference $|\delta\varphi|$ between two reference samples 12 (ref, ref), while Fig. 8B shows the measured phase difference $|\delta\varphi|$ between a reference sample 12 (ref) and a binding sample 12 (S+). The phase signal of different higher harmonics are identified by their order ("2nd, 3rd, 4th, 5th, 6th") for the second to sixth higher harmonic. The axes of each plot are the same as the ones of Fig. 7A, 7C.

The comparison of the data illustrates that the presence of the analyte may be reliably detected based on the measured phase difference $|\delta\varphi|$ for all of the higher harmonics starting from the 3rd higher harmonic, while the location of the critical point condition associated with the phase jump in the data differs for different higher harmonics.

Hence, by selecting a suitable combination of offset magnetic field strength $H_{DC}$ and excitation field amplitude $H_{AC}$ a precise measurement method may be implemented on the basis of measurement signals from different higher harmonics.

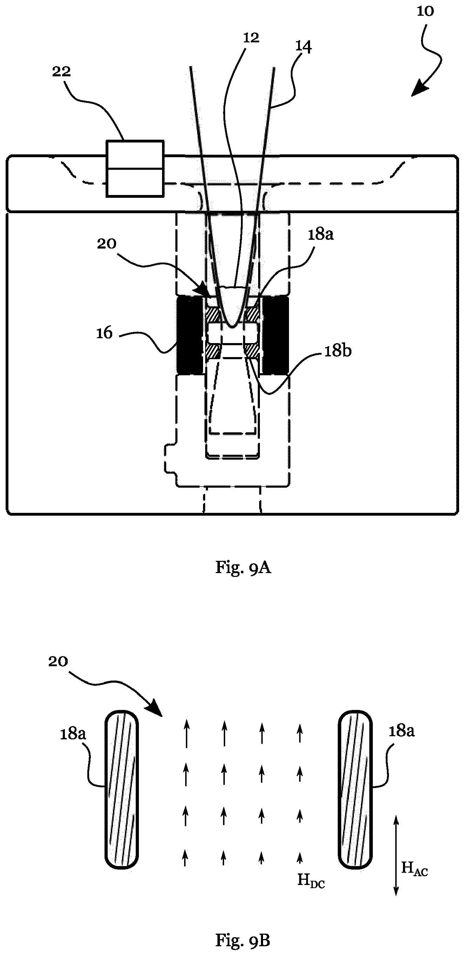

Fig. 9A illustrates a further system for a system 10 for measuring a (reference) sample 12 in a liquid container 14, which differs from the system illustrated in Fig. 6 by the addition of a permanent magnet 22. Fig. 9B illustrates a schematic example of the spatial distribution of the offset magnetic field strength $H_{DC}$ component induced by the permanent magnet 22 (magnitude of offset magnetic field indicated by differently sized arrows), which is oriented along the excitation field direction (indicated schematically by double-headed arrow in Fig. 9B), i.e. resulting from a projection of the respective magnetic field strengths onto the excitation field direction.

The permanent magnet 22 is arranged with an offset from the detection coil 18a, 18b and the excitation coil 16, in a direction along an excitation field direction (indicated schematically by double-headed arrow in Fig. 9B) of the excitation field generated by the excitation coil 16, and in a direction perpendicular to the excitation field direction. As a result, the offset magnetic field strength $H_{DC}$ in the measurement volume 20 associated with the one of the winding sections 18a may vary spatially as illustrated in the example in Fig. 9B.

The spatially varying offset magnetic field strength $H_{DC}$ induced by the permanent magnet 22 in the measurement volume 20 may therefore define an offset magnetic field gradient, which may lead to the critical point condition being fulfilled in a portion of a (reference) sample 12 20 for a given excitation field $H_{AC}$. The offset magnetic field gradient may reduce a requirement of sophistication related to the measurement of a modified property of the sample 12, as the critical point condition may be fulfilled in a broader range of critical offset magnetic fields. In some embodiments, the permanent magnet 22 may be the only offset magnetic field source, such that no direct current may be required for selecting the critical point condition.

For example, the system 10 may comprise a neodymium permanent magnet 22, which may induce magnetic field strengths in a range between about 5 mT to about 10 mT in the measurement volume 20. Hence, a component of the offset magnetic field $H_{DC}$ along the excitation field direction (i.e. the illustrated z-projection) may vary in a range of offset magnetic fields of about 3 mT over the (reference) sample 12 around a mean value of about 5 mT.

However, the permanent magnet 22 may also be combined with additional (in-)homogenous offset magnetic fields $H_{DC}$, e.g. generated with a magnetic field coil 16, in some embodiments.

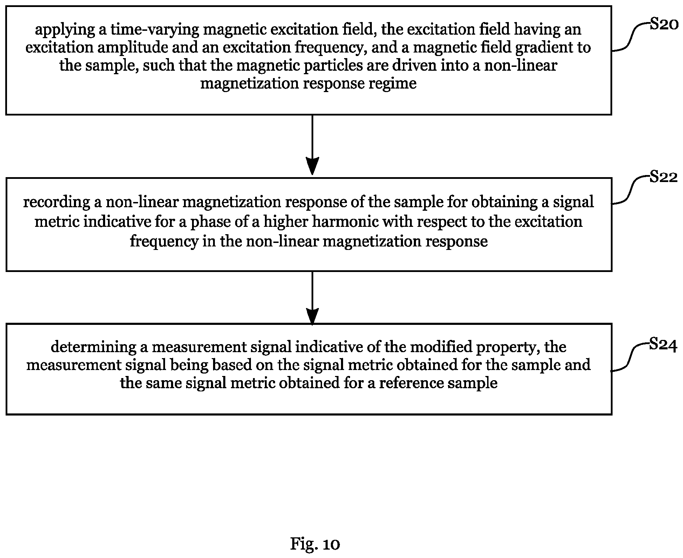

Fig. 10 illustrates an example of a method for measuring a modified property of a sample 12 using a magnetic field gradient. The method comprises applying a time-varying magnetic excitation field $H_{AC}(t)$, the excitation field having an excitation amplitude $H_{AC}$ and an excitation frequency $f_{AC}$, and a magnetic field gradient to the sample 12, such that the magnetic particles are driven into a non-linear magnetization response regime (S20), and recording a non-linear magnetization response of the sample 12 for obtaining a signal metric indicative for a phase of a higher harmonic with respect to the excitation frequency $f_{AC}$ in the non-linear magnetization response (S22). The method further comprises determining a measurement signal indicative of the modified property, the measurement signal being based on the signal metric obtained for the sample 12 and the same signal metric obtained for a reference sample 12 (S24).

The reference sample 12 does not feature the modified property, but also comprises the magnetic particles in suspension. The magnetic field gradient and the time-varying magnetic excitation field $H_{AC}(t)$ are selected such that a range of offset magnetic field strengths $H_{DC}$ of the magnetic field gradient overlaps a critical offset magnetic field range associated with a phase jump of the higher harmonic, when the sample 12 and/or the reference sample 12 is subjected to the excitation field $H_{AC}(t)$.

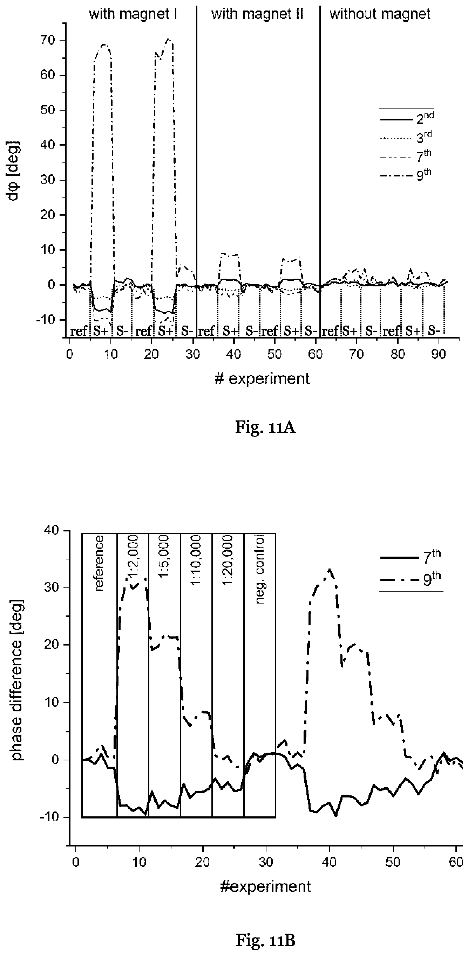

Fig. 11A, 11B illustrate raw data of preliminary measurements using a system 10 with a permanent magnet 22 inducing a magnetic field gradient in a measurement volume 20 as illustrated in Fig. 9A based on a method as illustrated in Fig. 10. The horizontal axis indicates an identifier associated with an experiment of a sequence of consecutive experiments conducted in chronological order, while the vertical axis illustrates a measured phase difference $|\delta\varphi|$ between a measurement of a sample 12 and a reference sample 12 (e.g. measured at the start of the sequence), wherein the phase difference $|\delta\varphi|$ is exemplarily shown for three different higher harmonics, as specified in the legend.

Each phase measurement is the result of a Fourier analysis of the non-linear magnetization response of the respective (reference) samples 12 to an applied time-varying magnetic excitation field $H_{AC}(t)$ at an excitation frequency $f_{AC}$ of 20 kHz and an excitation amplitude $H_{AC}$ of approximately 50 mT over a duration of approx. 10 ms, while it is affected by the offset magnetic field gradient of the permanent magnet 22 in the measurement volume 20.

The samples 12 were changed after a number of consecutive measurements of the phase difference $|\delta\varphi|$, wherein the respective samples 12 are indicated in the lower portion of Fig. 11A. In chronological order, the samples 12 were cycled between a reference sample 12 ("ref') containing a buffer solution, a binding sample 12 ("S+") containing a binding antibody in the buffer solution, and a non-binding sample 12 ("S-") containing a non-binding antibody in the buffer solution, as specified above.

After 30 measurements with the permanent magnet 22 in a first configuration ("with magnet I"), the orientation of the permanent magnet 22 was rotated by 90° towards a second configuration ("with magnet II"), which effectively results in a reduction of the component of the offset magnetic field strength $H_{DC}$ parallel to the excitation field direction in the measurement volume 20. After 60 measurements, the permanent magnet 22 was removed from the vicinity of the measurement volume 20 ("without magnet"), such that the offset magnetic field strength $H_{DC}$ substantially vanishes in the measurement volume 20.

For the first configuration ("with magnet I"), the phase of all but the third illustrated higher harmonics shows a clear signal which is distinguished both from a corresponding measurement of a reference sample 12 and a non-binding sample 12, while the phase difference is largest for the ninth ("9th") higher harmonic. For the second configuration, the measurement shows significantly reduced magnitude of the phase difference $|\delta\varphi|$ for all of the illustrated higher harmonics, but a presence can still be detected based on the phase difference $|\delta\varphi|$ measured for the ninth ("9th") higher harmonic. The Inventors attribute the sensitivity of the measurement based on the phase difference $|\delta\varphi|$ of the ninth ("9th") higher harmonic in the second configuration on the low offset magnetic field strength $H_{DC}$ for which the critical point condition is fulfilled for this higher harmonic. Accordingly, although the overlap with the respective critical offset magnetic field range is reduced, the measurement remains sensitive to the presence of the binding antibodies. In both configurations of the permanent magnet 22, the binding sample 12 ("S+") may be distinguished from the non-binding sample 12 ("S-") based on the measured phase difference $|\delta\varphi|$.

When the magnet is removed ("without magnet"), the method can no longer distinguish between a binding sample 12 ("S+") and the reference sample 12 ("ref") or the non-binding sample 12 ("S-") based on the measured phase difference $|\delta\varphi|$. This behavior is expected, as this configuration does not select a critical point condition for measuring the presence of the binding antibody in the sample 12 based on the resulting offset magnetic field $H_{DC}$ of substantially zero.

The skilled person will appreciate that the phase difference $|\delta\varphi|$ of the second ("2nd") higher harmonic equally shows a clear signal for the binding sample 12 ("S+") with respect to the non-binding sample 12 ("S-") and the reference sample 12 ("ref"), although no phase jump was visible in the data of Fig. 8A, 8B. The Inventors attribute the sensitivity of the phase difference $|\delta\varphi|$ of the second ("2nd") higher harmonic to a phase jump around zero offset magnetic field $H_{DC}$, predicted for all even higher harmonics, wherein the magnetic field gradient selects portions of the critical offset magnetic field range associated with this phase jump close to zero offset magnetic field $H_{DC}$. Hence, although the amplitude of the even higher harmonics substantially vanishes for zero offset magnetic field, the phase of the corresponding signal at comparatively low offset magnetic fields may still be sensitive to the presence of the binding antibody. However, as also visible in Fig. 11A, 11B and confirmed by the measurements displayed in Figs. 8A, 8B, a measurement signal associated with critical points (phase jumps) at non-zero offset magnetic field can achieve a sensitivity which is approximately an order of magnitude larger than the sensitivity obtained based on the critical point around zero offset magnetic field, which is measured for the second (even) higher harmonic. The phase difference $|\delta\varphi|$ of the second ("2nd") higher harmonic also substantially vanishes in the absence of the permanent magnet 22 ("without magnet").

Fig. 11B shows similar experiments as in Fig. 11A, but in addition to a reference sample 12 ("ref"), and a non-binding sample ("neg. control"), further binding samples ("1:2,000, 1:5,000, 1:10,000, 1:20,000") having different dilutions of the binding antibody solution are measured. The sample 12 with a dilution of 1:20,000 of antibody solution to buffer solution corresponds to an antibody concentration of 50 ng/ml, resulting in a sensitivity of about 2 BAU (binding antibody unit).

The data shows an approximate proportionality between antibody concentration and the measured phase difference $|\delta\varphi|$ for the binding sample 12, which may indicate a suitability of the method for quantitative characterization of antibody concentrations in the sample 12. Moreover, the method appears to discriminate between the reference/non-binding sample 12 and the binding sample 12 also at dilutions of the antibody solution of about 1:20,000 for the 7th higher harmonic.

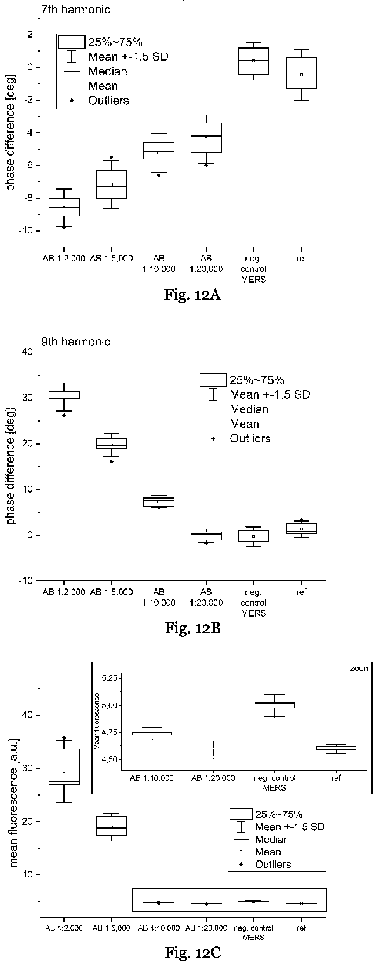

Figs. 12A, 12B illustrate the resulting median and mean values of the phase difference $|\delta\varphi|$ (horizontal axis) associated with the measurement sequences of a given reference/binding/non-binding sample 12 including statistical error bars for the measurement results of five consecutive measurements of each sample 12 (as specified in the horizontal axis). The statistical analysis confirms a sensitivity of the measurement to the binding antibodies up to dilutions of 1:10,000 for the 9th higher harmonic (Fig. 12A) and up to dilutions of 1:20,000 for the 7th higher harmonic (Fig. 12B).

The reduced sensitivity of the 9th higher harmonic may be a result of a decaying complex signal magnitude for higher orders of the higher harmonics and/or related to the limited sampling frequency of the system 10 used for the preliminary experiments, and may therefore likely be overcome with more sophisticated measurement hardware.

Fig. 12C illustrates a comparative measurement of identically prepared samples 12 as in Figs. 12A, 12B using fluorescence activated cell sorting (also known as FACS or "Flow cytometry"). The inset shows the mean fluorescence measured for each sample 12. Detection of antibodies was possible up to a dilution of 1:5000 with FACS-analysis.

As a result, the afore-mentioned methods may be exploited to implement a robust and fast sample 12 characterization method with a performance at least comparable to state of the art biochemical sensing methods, such as ELISA (Enzyme-linked Immunosorbent Assay) with a sensitivity of about 1-2 BAU. The results are particularly promising since the excitation amplitude $H_{AC}$ was not optimized to select a specific phase jump, but the selected condition only partially overlaps a critical offset magnetic field range of the 7th and 9th higher harmonics. Further improvements related to the sensitivity are therefore expected for lower excitation amplitudes $H_{AC}$ or larger offset magnetic fields $H_{DC}$, e.g. using stronger permanent magnets 22. On the other hand, the measurements in Figs. 11A-12B illustrate that the precise selection of the critical condition is not necessary for implementing a corresponding measurement. Rather, the presence of a non-zero offset magnetic field (gradient) $H_{DC}$ in combination with a suitable excitation amplitude to drive the magnetic nanoparticles into a non-linear magnetization response can suffice to measure the presence of the binding antibody, e.g. by selecting a "tail" of the critical offset magnetic field range.

The magnetic particles may be made of any suitable compound showing superparamagnetic behavior in a liquid suspension. Commonly, the magnetic core of magnetic nanoparticles comprises magnetite ($Fe_3O_4$) or maghemite ($\gamma$-$Fe_2O_3$) crystallites, which may feature a substantially fixed magnetization direction for crystallite diameters smaller than 100 nm. However, larger particle diameters may equally be used, e.g. as a result of larger nanoparticles being usually composed of multiple crystallites each showing essentially superparamagnetic properties. Such larger diameters may also be favored in some applications for facilitating a surface functionalization. Further, the diameter of the magnetic nanoparticles may also be increased due to the surface functionalization. For example, a functionalized magnetic nanoparticle may have a diameter of around 300 nm as discussed above. Accordingly, the skilled person will appreciate that the magnetic nanoparticles may have diameters of more than 100 nm or more than 300 nm in some embodiments, such as between 5 nm and 1000 nm or between 5 nm and 10 $\mu$m, and the magnetic particles may cluster because of binding, which may further increase the particle size.

Although the preferred embodiments have been described in conjunction with a preferred application related to biochemical sensing, other applications may equally be contemplated, wherein a modified property of the sample affects the rotational degree of freedom of the magnetic nanoparticles in suspension. For example, the method may be used to track a temperature of a sample 12 based on associated viscosity changes of a liquid suspension. As a further example, since the phase can be highly sensitive to offset magnetic field, the Inventors also tested the method for measuring changes of magnetic field, and found that the resolution for magnetic field changes can be on the order of 1 $\mu$T without significant optimization. Hence, the reference sample 12 may also be the sample 12 measured at a different point in time.

For sensing applications of magnetic fields, the magnetic (nano-)particles may also be smaller, such that the magnetic response may be significantly affected by Neel relaxation, since the magnetic particles do not necessarily have to be functionalized. Moreover, the skilled person will appreciate that for Neel relaxation the magnetic particles may also not perform a physical rotation. However, the phase jump will still be present for the critical point condition and may be used to measure changes of magnetic fields.

In this case, the excitation frequency may also be higher, e.g. more than 100 kHz, when compared to the common excitation frequencies for measuring particles in which the Brown relaxation effects results in a comparatively well-resolved inductive signal, such as between 1 kHz and 100 kHz.

The description of the preferred embodiments and the figures merely serve to illustrate the invention and the beneficial effects associated therewith, but should not be understood to imply any limitation. The scope of the invention is to be determined solely by the appended claims.

LIST OF REFERENCE SIGNS

| | |
|---|---|
| 10 | system |
| 12 | sample |
| 14 | sample container |
| 16 | excitation coil |
| 18a, 18b | upper and lower detection coil windings of gradiometer configuration |
| 20 | measurement volume |
| 22 | permanent magnet |

**Claims**

**1.** A method for measuring a modified property of a sample (12) comprising magnetic particles in suspension using time variable magnetic fields, wherein the method comprises:

a) applying a time-varying magnetic excitation field, the excitation field having an excitation amplitude and an excitation frequency, and a magnetic field gradient to the sample (12), such that the magnetic particles are driven into a non-linear magnetization response regime;

b) recording a non-linear magnetization response of the sample (12) for obtaining a signal metric indicative for a phase of a higher harmonic with respect to the excitation frequency in the non-linear magnetization response; and

c) determining a measurement signal indicative of the modified property, the measurement signal being based on the signal metric obtained for the sample (12) and the same signal metric obtained for a reference sample (12) without the modified property, the reference sample (12) also comprising the magnetic particles in suspension,

the method being **characterized in that**

the magnetic field gradient and the time-varying magnetic excitation field are selected such that a range of offset magnetic field strengths of the magnetic field gradient overlaps a critical offset magnetic field range associated with a phase jump of the higher harmonic, when the sample (12) and/or the reference sample (12) is subjected to the excitation field.

2. The method of claim 1, wherein the magnetic particles are functionalized to promote binding to a selected analyte, such that the modified property is a suspected presence of the selected analyte in the sample (12), wherein the analyte is in particular a biomolecule, preferably a protein, an antibody, a virus, RNA, DNA, a procariot, fungus, eucariot, or a portion thereof.

3. The method of claim 1 or 2, wherein the phase of the higher harmonic jumps by substantially 180° in the critical offset magnetic field range; and/or

- wherein the critical offset magnetic field range is centered on a crossing point of the real and imaginary components of the non-linear magnetization response associated with the higher harmonic.

4. The method of any one of the preceding claims, wherein the method comprises processing the non-linear magnetization response of the sample (12) for obtaining complex signal amplitudes of the higher harmonics, and wherein the signal metric is based on the complex signal amplitudes of the higher harmonic, and/or

- wherein processing the non-linear magnetization response comprises a Fourier transform of the non-linear magnetization response; and/or
- wherein the signal metric is proportional to or mathematically equivalent to a phase of the higher harmonic.

5. The method of any one of the preceding claims, wherein the excitation field induces a magnetic field having an excitation field direction, and wherein a parallel component of the magnetic field gradient along the excitation field direction varies over a measurement volume (20) associated with the sample (12) and/or the reference sample (12); and/or

- wherein applying the magnetic field gradient comprises applying the field of a permanent magnet (22).

6. The method of any one of the preceding claims, wherein the magnetic field gradient comprises offset magnetic field strengths larger than 2 mT; and/or

- wherein the excitation field has an excitation amplitude larger than 2 mT.

7. The method of any one of the preceding claims, wherein the measurement signal is mathematically equivalent or proportional to a difference between the signal metric obtained for the sample (12) and the signal metric obtained for the reference sample (12).

8. The method of any one of the preceding claims, wherein the method further comprises

a) applying the excitation field and the magnetic field gradient to the reference sample (12) without the modified property;

b) recording a non-linear magnetization response of the reference sample (12) for obtaining the signal metric indicative for the phase of the higher harmonic for the reference sample (12).

9. A method for measuring a modified property of a sample (12) comprising magnetic particles in suspension using time variable magnetic fields, wherein the method comprises:

a) applying a time-varying magnetic excitation field, the excitation field having an excitation amplitude and an excitation frequency, and a variable offset magnetic field to the sample (12), such that the magnetic particles are driven into a non-linear magnetization response regime;
b) recording a non-linear magnetization response of the sample (12) for obtaining a signal metric indicative for a phase of a higher harmonic with respect to the excitation frequency in the non-linear magnetization response; and
c) determining a measurement signal indicative of the modified property, the measurement signal being based on the signal metric obtained for the sample (12) and the signal metric obtained for a reference sample (12) without the modified property, the reference sample (12) also comprising the magnetic particles in suspension,

the method being **characterized in that**
the offset magnetic field is selected from a critical offset magnetic field range associated with a phase jump of the higher harmonic, when the sample (12) and/or the reference sample (12) is subjected to the excitation field.

10. The method of claim 9, wherein the offset magnetic field and/or the excitation amplitude are varied across the phase jump of the higher harmonic for the sample (12) and/or the reference sample (12), while recording the non-linear magnetization response of the sample (12) to the time-varying magnetic excitation field, wherein the measurement signal is preferably based on a resulting trace of the signal metric for the sample (12) and a corresponding trace of the signal metric for the reference sample (12).

11. A measurement system for measuring a modified property of a sample (12) comprising magnetic particles in suspension using time variable magnetic fields, wherein the system (10) comprises:

a) an inductive sensor associated with a measurement volume (20);
b) a magnetic field coil (16) for generating a time-varying magnetic field in the measurement volume (20);
c) an offset magnetic field source (16, 22) for generating an offset magnetic field in the measurement volume (20); and
d) a control system, wherein the control system is configured to:

- drive the magnetic field coil (16) to apply a time-varying magnetic excitation field to the measurement volume (20), the excitation field having an excitation amplitude and an excitation frequency, such that the magnetic particles of the sample (12) are driven into a non-linear magnetization response regime, and
- receive a non-linear magnetization response of the sample (12) from the inductive sensor for obtaining a signal metric indicative for a phase of a higher harmonic with respect to the excitation frequency in the non-linear magnetization response;
- determine a measurement signal indicative of the modified property, the measurement signal being based on the signal metric obtained for the sample (12) and the signal metric obtained for a reference sample (12) without the modified property, the reference sample (12) also comprising the magnetic particles in suspension,
- the measurement system (10) being **characterized in that** it is configured to select the offset magnetic field and the time-varying magnetic excitation field, such that the offset magnetic field is in a critical offset magnetic field range associated with a phase jump of the higher harmonic, when the sample (12) and/or the reference sample (12) is subjected to the excitation field.

12. The system (10) of claim 11, the magnetic field source comprising a variable magnetic field generator for generating the offset magnetic field in the critical offset magnetic field range;

- wherein the variable magnetic field generator is preferably coupled to the magnetic field coil (16) or to a second magnetic field coil; and/or
- wherein the variable magnetic field generator is preferably configured to vary the offset magnetic field over the critical offset magnetic field range for the sample (12) and the reference sample (12), while the system (10) records the non-linear magnetization response to the time-varying magnetic excitation field.

13. The system (10) of claim 11 or 12, wherein the magnetic field source is adapted to induce a magnetic field gradient in the measurement volume (20) and in particular comprises a permanent magnet arranged to induce a magnetic field gradient in the measurement volume (20).

14. The system (10) of any one of claims 11-13, wherein the inductive sensor comprises a measurement coil, in particular a gradiometer (18a, 18b).

15. A non-transitory medium comprising machine readable instructions, which, when executed by a processing system of a measurement system according to any one of claims 11-14, cause the processing system to implement a method according to any one of claims 1-10.

**Patentansprüche**

1. Verfahren zum Messen einer modifizierten Eigenschaft einer Probe (12), die schwebende magnetische Partikel umfasst, unter Verwendung zeitlich veränderlicher Magnetfelder, wobei das Verfahren Folgendes umfasst:

   a) Anlegen eines zeitlich veränderlichen magnetischen Anregungsfelds, wobei das Anregungsfeld eine Anregungsamplitude und eine Anregungsfrequenz aufweist, und eines Magnetfeldgradienten an die Probe (12), so dass die magnetischen Partikel in ein nichtlineares Magnetisierungsantwort-Regime getrieben werden;
   b) Aufzeichnen einer nichtlinearen Magnetisierungsantwort der Probe (12) zum Erhalten einer Signalmetrik, die indikativ für eine Phase einer höheren Harmonischen in Bezug auf die Anregungsfrequenz in der nichtlinearen Magnetisierungsantwort ist; und
   c) Bestimmen eines Messsignals, das indikativ für die modifizierte Eigenschaft ist, wobei das Messsignal auf der für die Probe (12) erhaltenen Signalmetrik und der gleichen Signalmetrik basiert, die für eine Referenzprobe (12) ohne die modifizierte Eigenschaft erhalten wird, wobei die Referenzprobe (12) auch die schwebenden magnetischen Partikel umfasst,

   wobei das Verfahren **dadurch gekennzeichnet ist, dass**
   der Magnetfeldgradient und das zeitlich veränderliche magnetische Anregungsfeld so ausgewählt werden, dass ein Bereich von Offset-Magnetfeldstärken des Magnetfeldgradienten einen kritischen Offset-Magnetfeldbereich überlappt, der mit einem Phasensprung der höheren Harmonischen assoziiert ist, wenn die Probe (12) und/oder die Referenzprobe (12) dem Anregungsfeld ausgesetzt wird.

2. Verfahren nach Anspruch 1, wobei die magnetischen Partikel funktionalisiert sind, um die Bindung an einen ausgewählten Analyten zu fördern, so dass die modifizierte Eigenschaft eine vermutete Anwesenheit des ausgewählten Analyten in der Probe (12) ist, wobei der Analyt insbesondere ein Biomolekül, vorzugsweise ein Protein, ein Antikörper, ein Virus, RNA, DNA, ein Prokariot, ein Pilz, Eukariot oder ein Teil davon ist.

3. Verfahren nach Anspruch 1 oder 2, wobei die Phase der höheren Harmonischen um im Wesentlichen 180° in dem kritischen Offset-Magnetfeldbereich springt; und/oder

   - wobei der kritische Offset-Magnetfeldbereich auf einem Kreuzungspunkt der realen und imaginären Komponenten der nichtlinearen Magnetisierungsantwort zentriert ist, die mit der höheren Harmonischen assoziiert ist.

4. Verfahren nach einem der vorhergehenden Ansprüche, wobei das Verfahren das Verarbeiten der nichtlinearen Magnetisierungsantwort der Probe (12) zum Erhalten komplexer Signalamplituden der höheren Harmonischen umfasst, und wobei die Signalmetrik auf den komplexen Signalamplituden der höheren Harmonischen basiert, und/oder

   - wobei das Verarbeiten der nichtlinearen Magnetisierungsantwort eine FourierTransformation der nichtlinearen Magnetisierungsantwort umfasst; und/oder
   - wobei die Signalmetrik proportional zu oder mathematisch äquivalent zu einer Phase der höheren Harmonischen ist.

5. Verfahren nach einem der vorhergehenden Ansprüche, wobei das Anregungsfeld ein Magnetfeld mit einer Anregungsfeldrichtung induziert, und wobei eine parallele Komponente des Magnetfeldgradienten entlang der Anregungsfeldrichtung über ein Messvolumen (20) variiert, das mit der Probe (12) und/oder der Referenzprobe (12) assoziiert ist; und/oder

   - wobei das Anlegen des Magnetfeldgradienten das Anlegen des Felds eines Permanentmagneten (22) umfasst.

6. Verfahren nach einem der vorhergehenden Ansprüche, wobei der Magnetfeldgradient Offset-Magnetfeldstärken von mehr als 2 mT umfasst; und/oder

   - wobei das Anregungsfeld eine Anregungsamplitude von mehr als 2 mT aufweist.

7. Verfahren nach einem der vorhergehenden Ansprüche, wobei das Messsignal mathematisch äquivalent oder proportional zu einer Differenz zwischen der für die Probe (12) erhaltenen Signalmetrik und der für die Referenzprobe (12) erhaltenen Signalmetrik ist.

8. Verfahren nach einem der vorhergehenden Ansprüche, wobei das Verfahren ferner Folgendes umfasst:

   a) Anlegen des Anregungsfelds und des Magnetfeldgradienten an die Referenzprobe (12) ohne die modifizierte Eigenschaft;
   b) Aufzeichnen einer nichtlinearen Magnetisierungsantwort der Referenzprobe (12) zum Erhalten der Signalmetrik, die indikativ für die Phase der höheren Harmonischen für die Referenzprobe (12) ist.

9. Verfahren zum Messen einer modifizierten Eigenschaft einer Probe (12), die schwebende magnetische Partikel umfasst, unter Verwendung zeitlich veränderlicher Magnetfelder, wobei das Verfahren Folgendes umfasst:

   a) Anlegen eines zeitlich veränderlichen magnetischen Anregungsfelds, wobei das Anregungsfeld eine Anregungsamplitude und eine Anregungsfrequenz aufweist, und eines Magnetfelds mit variablem Versatz an die Probe (12), so dass die magnetischen Partikel in ein nichtlineares Magnetisierungsantwort-Regime getrieben werden;
   b) Aufzeichnen einer nichtlinearen Magnetisierungsantwort der Probe (12) zum Erhalten einer Signalmetrik, die indikativ für eine Phase einer höheren Harmonischen in Bezug auf die Anregungsfrequenz in der nichtlinearen Magnetisierungsantwort ist; und
   c) Bestimmen eines Messsignals, das indikativ für die modifizierte Eigenschaft ist, wobei das Messsignal auf der für die Probe (12) erhaltenen Signalmetrik und der Signalmetrik basiert, die für eine Referenzprobe (12) ohne die modifizierte Eigenschaft erhalten wird, wobei die Referenzprobe (12) auch die schwebenden magnetischen Partikel umfasst,

   wobei das Verfahren **dadurch gekennzeichnet ist, dass**
   das Offset-Magnetfeld aus einem kritischen Offset-Magnetfeldbereich ausgewählt wird, der mit einem Phasensprung der höheren Harmonischen assoziiert ist, wenn die Probe (12) und/oder die Referenzprobe (12) dem Anregungsfeld ausgesetzt wird.

10. Verfahren nach Anspruch 9, wobei das Offset-Magnetfeld und/oder die Anregungsamplitude über den Phasensprung der höheren Harmonischen für die Probe (12) und/oder die Referenzprobe (12) variiert werden, während die nichtlineare Magnetisierungsantwort der Probe (12) auf das zeitlich veränderliche magnetische Anregungsfeld aufgezeichnet wird, wobei das Messsignal vorzugsweise auf einer resultierenden Abtastung der Signalmetrik für die Probe (12) und einer entsprechenden Abtastung der Signalmetrik für die Referenzprobe (12) basiert.

11. Messsystem zum Messen einer modifizierten Eigenschaft einer Probe (12), die schwebende magnetische Partikel umfasst, unter Verwendung zeitlich veränderlicher Magnetfelder, wobei das System (10) Folgendes umfasst:

   a) einen induktiven Sensor, der mit einem Messvolumen (20) assoziiert ist;
   b) eine Magnetfeldspule (16) zum Erzeugen eines zeitlich veränderlichen Magnetfelds in dem Messvolumen (20);
   c) eine Offset-Magnetfeldquelle (16, 22) zum Erzeugen eines Offset-Magnetfelds in dem Messvolumen (20); und
   d) ein Steuersystem, wobei das Steuersystem eingerichtet ist zum:

      - Antreiben der Magnetfeldspule (16), um ein zeitlich veränderliches magnetisches Anregungsfeld an das Messvolumen (20) anzulegen, wobei das Anregungsfeld eine Anregungsamplitude und eine Anregungsfrequenz aufweist, so dass die magnetischen Partikel der Probe (12) in ein nichtlineares Magnetisierungsantwort-Regime getrieben werden, und
      - Empfangen einer nichtlinearen Magnetisierungsantwort der Probe (12) von dem induktiven Sensor zum Erhalten einer Signalmetrik, die indikativ für eine Phase einer höheren Harmonischen in Bezug auf die Anregungsfrequenz in der nichtlinearen Magnetisierungsantwort ist;

- Bestimmen eines Messsignals, das indikativ für die modifizierte Eigenschaft ist, wobei das Messsignal auf der für die Probe (12) erhaltenen Signalmetrik und der Signalmetrik basiert, die für eine Referenzprobe (12) ohne die modifizierte Eigenschaft erhalten wird, wobei die Referenzprobe (12) auch die schwebenden magnetischen Partikel umfasst,
- wobei das Messsystem (10) **dadurch gekennzeichnet ist, dass** es eingerichtet ist, das Offset-Magnetfeld und das zeitlich veränderliche magnetische Anregungsfeld so auszuwählen, dass das Offset-Magnetfeld in einem kritischen Offset-Magnetfeldbereich liegt, der mit einem Phasensprung der höheren Harmonischen assoziiert ist, wenn die Probe (12) und/oder die Referenzprobe (12) dem Anregungsfeld ausgesetzt wird.

12. System (10) nach Anspruch 11, wobei die Magnetfeldquelle einen Generator variabler Magnetfelder zum Erzeugen des Offset-Magnetfelds in dem kritischen Offset-Magnetfeldbereich umfasst;

- wobei der Generator variabler Magnetfelder vorzugsweise mit der Magnetfeldspule (16) oder mit einer zweiten Magnetfeldspule gekoppelt ist; und/oder
- wobei der Generator variabler Magnetfelder vorzugsweise eingerichtet ist, das Offset-Magnetfeld über den kritischen Offset-Magnetfeldbereich für die Probe (12) und die Referenzprobe (12) zu variieren, während das System (10) die nichtlineare Magnetisierungsantwort auf das zeitlich veränderliche magnetische Anregungsfeld aufzeichnet.

13. System (10) nach Anspruch 11 oder 12, wobei die Magnetfeldquelle eingerichtet ist, einen Magnetfeldgradienten in dem Messvolumen (20) zu induzieren, und insbesondere einen Permanentmagneten umfasst, der eingerichtet ist, einen Magnetfeldgradienten in dem Messvolumen (20) zu induzieren.

14. System (10) nach einem der Ansprüche 11-13, wobei der induktive Sensor eine Messspule, insbesondere ein Gradiometer (18a, 18b), umfasst.

15. Nichtflüchtiges Medium, das maschinenlesbare Anweisungen umfasst, die, wenn sie von einem Verarbeitungssystem eines Messsystems nach einem der Ansprüche 11-14 ausgeführt werden, das Verarbeitungssystem veranlassen, ein Verfahren nach einem der Ansprüche 1-10 zu implementieren.

**Revendications**

1. Procédé de mesure d'une propriété modifiée d'un échantillon (12) comprenant des particules magnétiques en suspension à l'aide de champs magnétiques variables dans le temps, le procédé comprenant :

a) l'application d'un champ d'excitation magnétique variable dans le temps, le champ d'excitation ayant une amplitude d'excitation et une fréquence d'excitation, et d'un gradient de champ magnétique à l'échantillon (12), de telle sorte que les particules magnétiques sont excitées dans un régime de réponse de magnétisation non linéaire ;
b) l'enregistrement d'une réponse de magnétisation non linéaire de l'échantillon (12) pour obtenir une métrique de signal indiquant une phase d'une harmonique supérieure par rapport à la fréquence d'excitation dans la réponse de magnétisation non linéaire ; et
c) la détermination d'un signal de mesure indicatif de la propriété modifiée, le signal de mesure étant basé sur la métrique de signal obtenue pour l'échantillon (12) et la même métrique de signal obtenue pour un échantillon de référence (12) sans la propriété modifiée, l'échantillon de référence (12) comprenant en outre les particules magnétiques en suspension,

le procédé étant **caractérisé en ce que**
le gradient de champ magnétique et le champ d'excitation magnétique variable dans le temps sont sélectionnés de sorte qu'une plage d'intensités de champ magnétique décalées du gradient de champ magnétique chevauche une plage de champ magnétique décalée critique associée à un saut de phase de l'harmonique supérieure, lorsque l'échantillon (12) et/ou l'échantillon de référence (12) est exposé au champ d'excitation.

2. Procédé selon la revendication 1, dans lequel les particules magnétiques sont fonctionnalisées pour favoriser la liaison à un analyte sélectionné, de telle sorte que la propriété modifiée est une présence suspectée de l'analyte sélectionné dans l'échantillon (12), dans lequel l'analyte est en particulier une biomolécule, de préférence une protéine, un anticorps, un virus, un ARN, un ADN, un procaryote, un champignon, un eucaryote ou une partie de ceux-ci.

3. Procédé selon la revendication 1 ou 2, dans lequel la phase de l'harmonique supérieure saute de sensiblement 180° dans la plage critique de champ magnétique décalé ; et/ou

 - dans lequel la plage critique de champ magnétique décalé est centrée sur un point de croisement des composantes réelle et imaginaire de la réponse de magnétisation non linéaire associée à l'harmonique supérieure.

4. Procédé selon l'une quelconque des revendications précédentes, dans lequel le procédé comprend le traitement de la réponse de magnétisation non linéaire de l'échantillon (12) pour obtenir des amplitudes de signal complexes des harmoniques supérieures, et dans lequel la métrique de signal est basée sur les amplitudes de signal complexes de l'harmonique supérieure, et/ou

 - dans lequel le traitement de la réponse de magnétisation non linéaire comprend une transformée de Fourier de la réponse de magnétisation non linéaire ; et/ou
 - dans lequel la métrique du signal est proportionnelle ou mathématiquement équivalente à une phase de l'harmonique supérieure.

5. Procédé selon l'une quelconque des revendications précédentes, dans lequel le champ d'excitation induit un champ magnétique ayant une direction de champ d'excitation, et dans lequel une composante parallèle du gradient de champ magnétique le long de la direction du champ d'excitation varie sur un volume de mesure (20) associé au l'échantillon (12) et/ou l'échantillon de référence (12) ; et/ou

 - dans lequel l'application du gradient de champ magnétique comprend l'application du champ d'un aimant permanent (22).

6. Procédé selon l'une quelconque des revendications précédentes, dans lequel le gradient de champ magnétique comprend des intensités de champ magnétique décalé supérieures à 2 mT ; et/ou

 - dans lequel le champ d'excitation a une amplitude d'excitation supérieure à 2 mT.

7. Procédé selon l'une quelconque des revendications précédentes, dans lequel le signal de mesure est mathématiquement équivalent ou proportionnel à une différence entre la métrique du signal obtenue pour l'échantillon (12) et la métrique du signal obtenue pour l'échantillon de référence (12).

8. Procédé selon l'une quelconque des revendications précédentes, le procédé comprenant en outre

 a) l'application du champ d'excitation et du gradient de champ magnétique à l'échantillon de référence (12) sans la propriété modifiée ;
 b) l'enregistrement d'une réponse de magnétisation non linéaire de l'échantillon de référence (12) pour obtenir la métrique de signal indiquant la phase de l'harmonique supérieure pour l'échantillon de référence (12).

9. Procédé de mesure d'une propriété modifiée d'un échantillon (12) comprenant des particules magnétiques en suspension à l'aide de champs magnétiques variables dans le temps, le procédé comprenant :

 a) l'application d'un champ d'excitation magnétique variable dans le temps, le champ d'excitation ayant une amplitude d'excitation et une fréquence d'excitation, et d'un champ magnétique décalé variable à l'échantillon (12), de telle sorte que les particules magnétiques sont excitées dans un régime de réponse de magnétisation non linéaire ;
 b) l'enregistrement d'une réponse de magnétisation non linéaire de l'échantillon (12) pour obtenir une métrique de signal indiquant une phase d'une harmonique supérieure par rapport à la fréquence d'excitation dans la réponse de magnétisation non linéaire ; et
 c) la détermination d'un signal de mesure indiquant la propriété modifiée, le signal de mesure étant basé sur la métrique de signal obtenue pour l'échantillon (12) et la métrique de signal obtenue pour un échantillon de référence (12) sans la propriété modifiée, l'échantillon de référence (12) comprenant en outre les particules magnétiques en suspension,

le procédé étant **caractérisé en ce que**

le champ magnétique décalé est sélectionné dans une plage critique de champ magnétique décalé associée à un saut de phase de l'harmonique supérieure, lorsque l'échantillon (12) et/ou l'échantillon de référence (12) est exposé au champ d'excitation.

10. Procédé selon la revendication 9, dans lequel le champ magnétique décalé et/ou l'amplitude d'excitation varient à travers le saut de phase de l'harmonique supérieure pour l'échantillon (12) et/ou l'échantillon de référence (12), tout en enregistrant la réponse de magnétisation non linéaire de l'échantillon (12) au champ d'excitation magnétique variable dans le temps, le signal de mesure étant de préférence basé sur une trace résultante de la métrique de signal pour l'échantillon (12) et une trace correspondante de la métrique de signal pour l'échantillon de référence (12).

11. Système de mesure pour mesurer une propriété modifiée d'un échantillon (12) comprenant des particules magnétiques en suspension à l'aide de champs magnétiques variables dans le temps, le système (10) comprenant :

a) un capteur inductif associé à un volume de mesure (20) ;
b) une bobine de champ magnétique (16) pour générer un champ magnétique variable dans le temps dans le volume de mesure (20) ;
c) une source de champ magnétique décalé (16, 22) pour générer un champ magnétique décalé dans le volume de mesure (20) ; et
d) un système de commande, le système de commande étant configuré pour :

- exciter la bobine de champ magnétique (16) pour appliquer un champ d'excitation magnétique variable dans le temps au volume de mesure (20), le champ d'excitation ayant une amplitude d'excitation et une fréquence d'excitation, de telle sorte que les particules magnétiques de l'échantillon (12) sont excitées dans un régime de réponse de magnétisation non linéaire, et
- recevoir une réponse de magnétisation non linéaire de l'échantillon (12) depuis le capteur inductif pour obtenir une métrique de signal indiquant une phase d'harmonique supérieure par rapport à la fréquence d'excitation dans la réponse de magnétisation non linéaire ;
- déterminer un signal de mesure indiquant la propriété modifiée, le signal de mesure étant basé sur la métrique de signal obtenue pour l'échantillon (12) et la métrique de signal obtenue pour un échantillon de référence (12) sans la propriété modifiée, l'échantillon de référence (12) comprenant en outre les particules magnétiques en suspension,
- le système de mesure (10) étant **caractérisé en ce qu'**il est configuré pour sélectionner le champ magnétique décalé et le champ magnétique d'excitation variable dans le temps, de telle sorte que le champ magnétique décalé soit dans une plage critique de champ magnétique décalé associée à un saut de phase de l'harmonique supérieure, lorsque l'échantillon (12) et/ou l'échantillon de référence (12) est soumis au champ d'excitation.

12. Système (10) selon la revendication 11, la source de champ magnétique comprenant un générateur de champ magnétique variable pour générer le champ magnétique décalé dans la plage critique de champ magnétique décalé ;

- dans lequel le générateur de champ magnétique variable est de préférence couplé à la bobine de champ magnétique (16) ou à une deuxième bobine de champ magnétique ; et/ou
- dans lequel le générateur de champ magnétique variable est de préférence configuré pour faire varier le champ magnétique décalé dans la plage critique de champ magnétique décalé pour l'échantillon (12) et l'échantillon de référence (12), tandis que le système (10) enregistre la réponse de magnétisation non linéaire au champ d'excitation magnétique variable dans le temps.

13. Système (10) selon la revendication 11 ou 12, dans lequel la source de champ magnétique est adaptée pour induire un gradient de champ magnétique dans le volume de mesure (20) et comprend notamment un aimant permanent agencé pour induire un gradient de champ magnétique dans le volume de mesure (20).

14. Système (10) selon l'une quelconque des revendications 11 à 13, dans lequel le capteur inductif comprend une bobine de mesure, en particulier un gradiomètre (18a, 18b).

15. Support non transitoire comprenant des instructions lisibles par machine qui, lorsqu'elles sont exécutées par un système de traitement d'un système de mesure selon l'une quelconque des revendications 11 à 14, amènent le système de traitement à mettre en oeuvre un procédé selon l'une quelconque des revendications 1 à 10.

(a)

(b)

M

M(t)

H

t

Fourier transform

(c)

(d)

t

A

$H_{AC}(t)$

$H_{AC}(t)+H'_{DC}$

$H_{DC}$

$H'_{DC}$

H

$H'_{DC}$

$1f_1$  $2f_1$  $3f_1$  $4f_1$  $5f_1$  $6f_1$  $7f_1$  $8f_1$  $9f_1$   f

(e)

dip

A

$1f_1$  $2f_1$  $3f_1$  $4f_1$  $5f_1$  $6f_1$  $7f_1$  $8f_1$  $9f_1$   f

Fig. 1

Fig. 2A

Fig. 2B

Fig. 3A

Fig. 3B

applying a time-varying magnetic excitation field, the excitation field having an excitation amplitude and an excitation frequency, and a offset magnetic field to the sample, such that the magnetic particles are driven into a non-linear magnetization response regime — S10

recording a non-linear magnetization response of the sample for obtaining a signal metric indicative for a phase of a higher harmonic with respect to the excitation frequency in the non-linear magnetization response — S12

determining a measurement signal indicative of the modified property, the measurement signal being based on the signal metric obtained for the sample and the signal metric obtained for a reference sample — S14

Fig. 4

Fig. 5

Fig. 6

Fig. 7A

Fig. 7B

Fig. 7C

Fig. 7D

Fig. 8A

Fig. 8B

Fig. 9A

Fig. 9B

applying a time-varying magnetic excitation field, the excitation field having an excitation amplitude and an excitation frequency, and a magnetic field gradient to the sample, such that the magnetic particles are driven into a non-linear magnetization response regime ⟋ S20

recording a non-linear magnetization response of the sample for obtaining a signal metric indicative for a phase of a higher harmonic with respect to the excitation frequency in the non-linear magnetization response ⟋ S22

determining a measurement signal indicative of the modified property, the measurement signal being based on the signal metric obtained for the sample and the same signal metric obtained for a reference sample ⟋ S24

Fig. 10

Fig. 11A

Fig. 11B

Fig. 12A

Fig. 12B

Fig. 12C

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021212144 A1

**Non-patent literature cited in the description**

- **WU et al.** One-Step, Wash-free, Nanoparticle Clustering-Based Magnetic nanoparticle Spectroscopy Bioassay Method for Detection of SARS-CoV-2 Spike and Nucleocapsid Proteins in the Liquid Phase. *ACS Appl. Mater. Interfaces,* 2021, vol. 13, 44136-44146